# DEMANDE DE BREVET EUROPEEN

(11) **EP 1 568 355 A1**
(43) Date de publication de la demande: **31.08.2005**
(21) Numéro de dépôt: 05290425.7
(22) Date de dépôt: 24.02.2005
(51) Int. Cl.: A61K 7/13, C07C 217/76

(54) **Para-phénylènediamine secondaire n-alkylhydroxylée ortho- et/ou méta-substituée, composition de teinture des fibres kératiniques contenant une telle para-phénylènediamine, procédés mettant en oeuvre cette composition et utilisations**

(30) Priorité: 27.02.2004 FR 0402019
(71) Demandeur: L'OREAL, 75008 Paris (FR)
(72) Inventeur: Sabelle, Stéphane, 75005 Paris (FR)
(74) Mandataire: Dossmann, Gérard

(57) **Abrégé**

La présente demande concerne de nouvelles para-phénylènediamines secondaires N-alkylhydroxylées, une composition pour la teinture des fibres kératiniques, et en particulier les fibres kératiniques humaines telles que les cheveux, contenant, dans un milieu approprié pour la teinture, au moins une para-phénylènediamine secondaire N-alkylhydroxylée ortho et/ou méta substituée, un procédé de teinture des fibres kératiniques qui consiste à appliquer cette composition ainsi que les utilisations de cette composition en particulier sous la forme d'un "kit" de teinture.

## Description

La présente invention a pour objet une nouvelle famille de para-phénylènediamines secondaires N-alkylhydroxylées ortho substituée et/ou méta substituée et leur utilisation pour la teinture des fibres kératiniques, en particulier des fibres kératiniques humaines telles que les cheveux.

Il est connu de teindre les fibres kératiniques et en particulier les cheveux humains avec des compositions tinctoriales contenant des précurseurs de colorants d'oxydation, appelés généralement bases d'oxydation, tels que des ortho- ou para-phénylènediamines, des ortho- ou para-aminophénols et des composés hétérocycliques. Ces bases d'oxydation sont des composés incolores ou faiblement colorés qui, associés à des produits oxydants, peuvent donner naissance à des composés colorés par un processus de condensation oxydative.

On sait également que l'on peut faire varier les nuances obtenues avec ces bases d'oxydation en les associant à des coupleurs ou modificateurs de coloration, ces derniers étant choisis notamment parmi les méta-diaminobenzènes, les méta-aminophénols, les méta-diphénols et certains composés hétérocycliques tels que des composés indoliques.

La variété des molécules mises en jeu au niveau des bases d'oxydation et des coupleurs permet l'obtention d'une riche palette de couleurs.

La coloration dite "permanente" obtenue grâce à ces colorants d'oxydation, doit par ailleurs satisfaire un certain nombre d'exigences. Ainsi, elle doit être sans inconvénient sur le plan toxicologique, elle doit permettre d'obtenir des nuances dans l'intensité souhaitée et présenter une bonne tenue face aux agents extérieurs tels que la lumière, les intempéries, le lavage, les ondulations permanentes, la transpiration et les frottements.

Les colorants doivent également permettre de couvrir les cheveux blancs, et être enfin les moins sélectifs possibles, c'est-à-dire permettre d'obtenir des écarts de coloration les plus faibles possibles tout au long d'une même fibre kératinique, qui est en général différemment sensibilisée (c'est-à-dire abîmée) entre sa pointe et sa racine.

De manière surprenante et avantageuse, la Demanderesse vient de découvrir qu'il est possible d'obtenir de nouvelles compositions pour la teinture des fibres kératiniques, en particulier des fibres kératiniques humaines telles que les cheveux, capables de conduire à des colorations aux nuances variées, puissantes, esthétiques, peu sélectives et résistant bien aux diverses agressions que peuvent subir les fibres, en utilisant au moins une para-phénylènediamine secondaire N-alkylhydroxylées ortho substituée et/ou méta-substituée.

En outre, ces compositions présentent un bon profil toxicologique.

Un premier objet de l'invention concerne une famille de para-phénylènediamines secondaires N-alkylhydroxylées ortho substituées et/ou méta substituées, leurs procédés de synthèse et leurs utilisations en particulier pour la teinture des fibres kératiniques, en particulier les fibres kératiniques humaines telles que les cheveux.

L'invention a aussi pour objet une composition contenant au moins une para-phénylènediamine secondaire N-alkylhydroxylée ortho substituée et/ou méta substituée, les procédés de teinture mettant en oeuvre cette composition, les utilisations de la composition selon la présente invention pour la teinture des fibres kératiniques, en particulier les fibres kératiniques humaines telles que les cheveux et en particulier, les dispositifs à plusieurs compartiments ou "kits" de teinture.

La composition de la présente invention permet en particulier d'obtenir une coloration des fibres kératiniques très puissante, peu sélective et tenace, en particulier à la lumière, tout en évitant la dégradation de ces fibres.

D'autres caractéristiques, aspects, objets et avantages de la présente invention apparaîtront encore plus clairement à la lecture de la description et des exemples qui suivent.

Dans le cadre de la présente invention, on entend par alkyle, un radical linéaire ou ramifié en C₁-C₁₀, par exemple les radicaux linéaires ou ramifiés suivants : méthyle, éthyle, propyle, butyle, pentyle, hexyle, octyle...

Les nouvelles para-phénylènediamines secondaires N-alkylhydroxylées ortho substituées et/ou méta substituées selon la présente invention sont des composés de formule générale (I) : 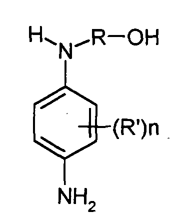 dans laquelle :
le radical R représente un radical alkylène en C₃-C₁₀, linéaire ou ramifié, éventuellement substitué par un ou plusieurs groupements choisis parmi les groupements amino, monoalkylamino, dialkylamino, alkylcarbonyle, amido, monoalkylaminocarbonyle, dialkylaminocarbonyle, éventuellement interrompu par un ou plusieurs atomes choisis parmi l'azote et l'oxygène,
le radical R' représente un radical choisi parmi les radicaux alkyle, alcoxy, hydroxyalcoxy, alcoxyalkyle, monohydroxyalkyle, polyhydroxyalkyle et l'atome de chlore,
n est un entier de 1 à 4.

Selon un premier mode préféré, le radical R représente un radical alkylène linéaire ou ramifié en C₃-C₈, de préférence en C₃-C₆.

Selon un deuxième mode préféré, le radical R représente un radical alkylène linéaire ou ramifié en C₃-C₆, de préférence en C₃-C₄ interrompu par un atome d'azote ou d'oxygène.

De manière préférée, le radical R' est un radical choisi parmi les groupes alkyles en C₁-C₃ et alcoxy en C₁-C₃, de manière encore plus préférée il s'agit d'un groupe méthyle.

De manière préférée n est égal à 1 ou 2, de manière encore plus préférée n est égal à 1.

Les composés de formule (I) peuvent se présenter sous forme libre ou sous forme de sels, tel que les sels d'addition avec un acide de préférence choisis parmi les chlorhydrates, les bromhydrates, les sulfates, les citrates, les succinates, les tartrates, les lactates, les tosylates, les benzènesulfonates, les phosphates et les acétates.

Quelques composés de formule (I) préférés sont présentés dans le tableau qui suit :

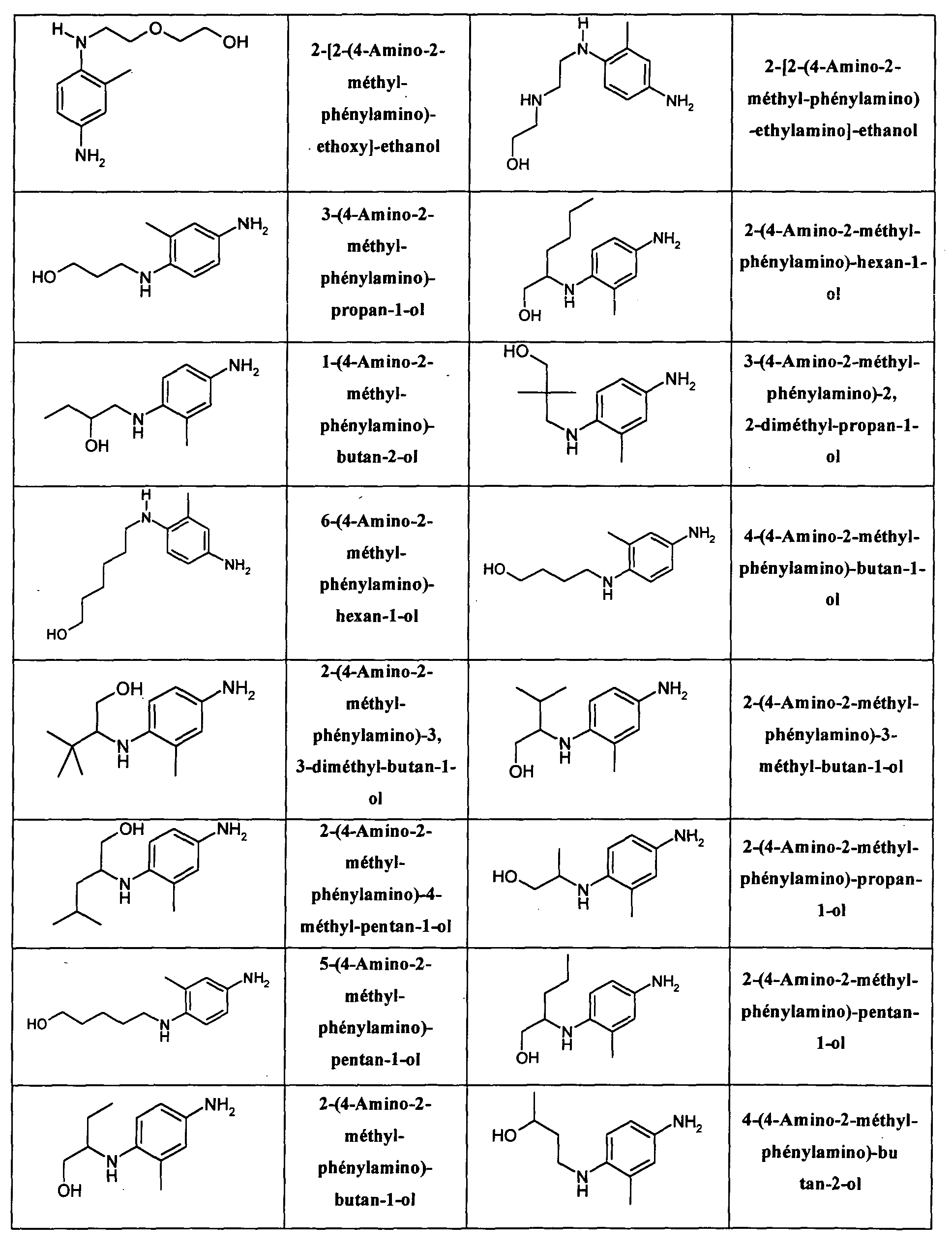

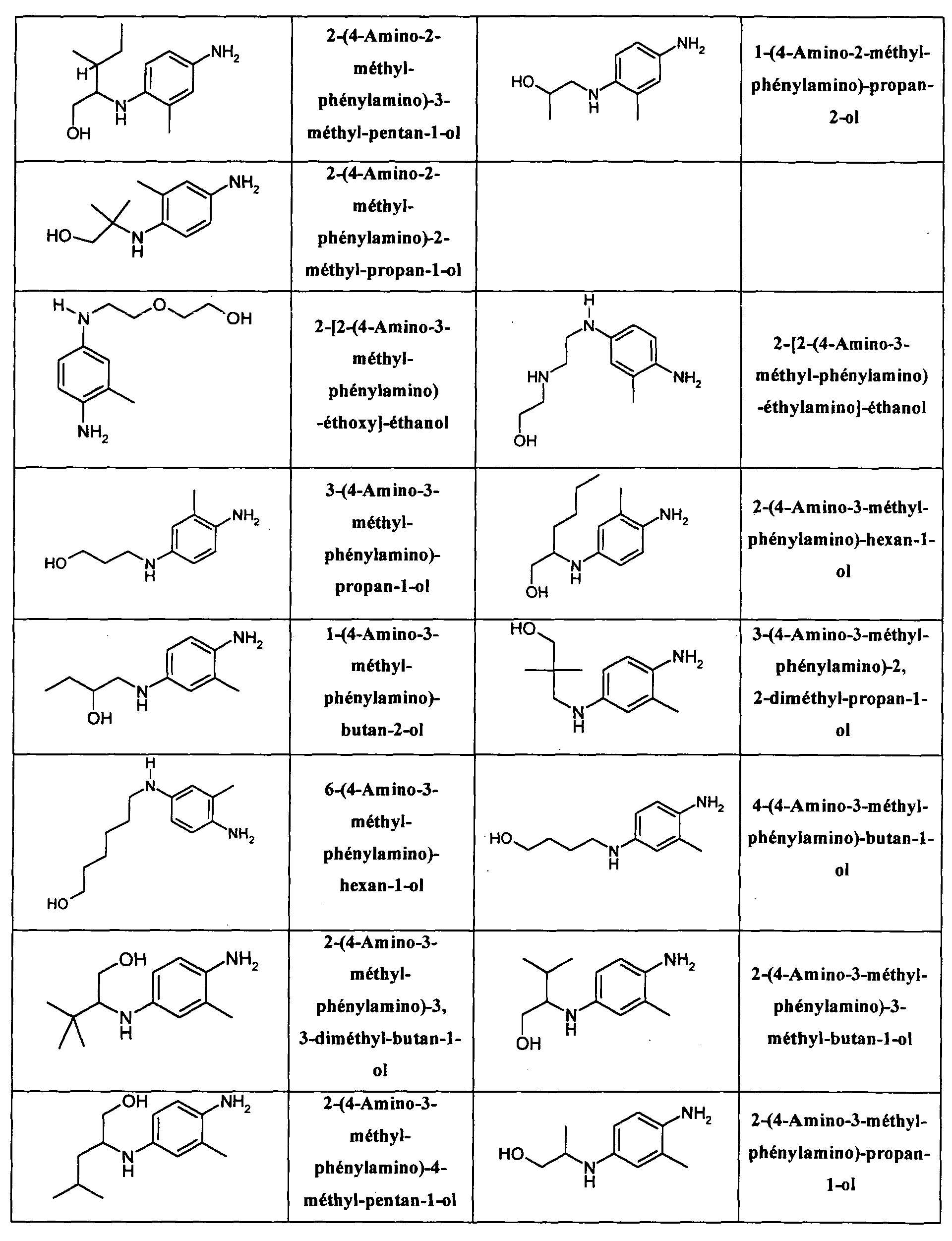

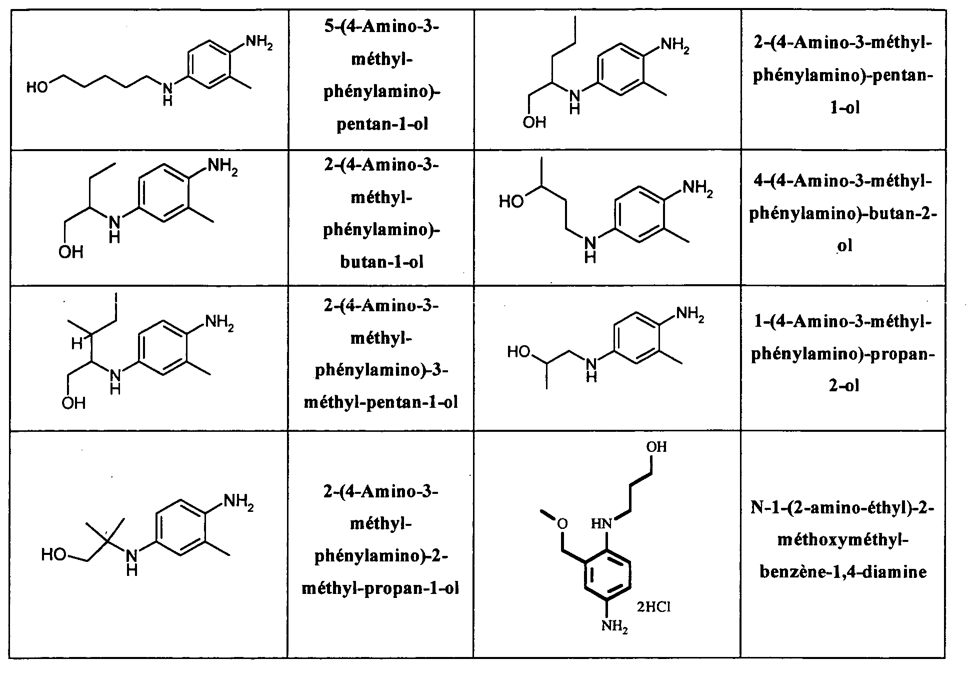

Les composés (I) selon la présente demande peuvent être préparés de façon générale suivant une méthode qui comprend les étapes suivantes : obtention d'un composé 4(N-alkylhydroxy) nitrobenzène par substitution nucléophile d'un halogène ou d'un radical benzyloxy par une amine hydroxylée de formule HOR-NH₂ (R étant tel que défini ci-dessus) en présence d'une base, puis réduction du groupement nitro du composé 4(N-alkylhydroxylé)nitrobenzène obtenu pour obtenir le composé de formule (I) :

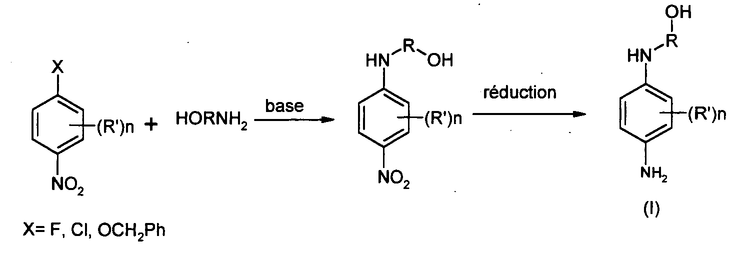

La première étape de synthèse est décrite dans les documents Synthesis 1990 (12), 1147-1148 et Synth Commun 1990, 20(22), 3537-3543.

La deuxième étape est une étape de réduction classique par exemple en effectuant une réaction d'hydrogénation par catalyse hétérogène en présence de Pd/C, Pd(II)/C, Nickel de Raney ou encore en effectuant une réaction de réduction par un métal, par exemple par du zinc, fer, étain. (Advanced Organic Chemistry, 4th edition, 1992, J. MARCH, WILEY Interscience ; Reduction in Organic Chemistry, M.Hudlicky, 1983, Ellis Honwood series Chemical Science).

La présente demande concerne aussi les composés nitrés de formule (II) et les procédés de préparation des composés para-phénylènediamines secondaires de formule (I), dans lesquels on effectue une étape de réduction du composé nitré correspondant, le « composé nitré correspondant » étant le composé de formule (I) dans lequel le groupe amino en para du groupe NHROH est remplacé par un groupe nitro.

Ces composés correspondent à la formule : 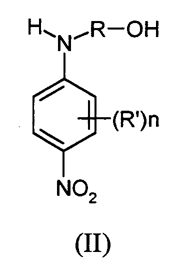 dans laquelle :
le radical R représente un radical alkylène en C₃-C₁₀, linéaire ou ramifié, éventuellement substitué par un ou plusieurs groupements choisis parmi les groupements amino, monoalkylamino, dialkylamino, alkylcarbonyle, amido, monoalkylaminocarbonyle, dialkylaminocarbonyle, éventuellement interrompu par un ou plusieurs atomes choisis parmi l'azote et l'oxygène,
le radical R' représente un radical choisi parmi les radicaux alkyle, alcoxy, hydroxyalcoxy, alcoxyalkyle, monohydroxyalkyle, polyhydroxyalkyle et l'atome de chlore,
n est un entier de 1 à 4.

La présente demande concerne également les utilisations des composés de formule (I) selon la présente demande, et en particulier, l'utilisation des composés de formule (I) selon la présente demande, pour la teinture des fibres, de préférence les fibres kératiniques telles que les cheveux.

La présente demande concerne aussi une composition cosmétique pour la teinture des fibres, de préférence les fibres kératiniques telles que les cheveux, comprenant, dans un milieu approprié pour la teinture, au moins un composé de formule générale (I).

De préférence, la concentration du composé de formule générale (I) est comprise entre 0,0001 et 20%, de préférence entre 0,005 à 6% en poids par rapport au poids total de la composition.

La présente demande concerne également l'utilisation d'une composition cosmétique comprenant, dans un milieu approprié pour la teinture, au moins un composé de formule générale (I), pour la teinture des fibres, de préférence les fibres kératiniques telles que les cheveux.

Le milieu approprié pour la teinture est avantageusement constitué par de l'eau ou par un mélange d'eau et d'au moins un solvant organique tel que, par exemple, les alcools inférieurs en C₁-C₄, ramifiés ou non, tels que l'éthanol et l'isopropanol ; les polyols et éthers de polyols comme le 2-butoxyéthanol, le propylèneglycol, le monométhyléther de propylèneglycol, le monoéthyléther et le monométhyléther du diéthylèneglycol, le glycérol ainsi que les alcools aromatiques comme l'alcool benzylique ou le phénoxyéthanol, et leurs mélanges.

Avantageusement, la composition cosmétique comprend au moins un adjuvant cosmétique choisi dans le groupe formé par les agents anti-oxydants, les agents de pénétration, les agents séquestrants, les parfums, les tampons, les agents dispersants, les tensioactifs, les agents conditionneurs, les agents filmogènes, les polymères, les céramides, les agents conservateurs, les agents nacrants ou opacifiants, les vitamines ou provitamines.

Les adjuvants ci-dessus sont en général présents en quantité comprise pour chacun d'eux entre 0,01 et 20 % en poids par rapport au poids de la composition.

La composition selon l'invention contient de préférence au moins un précurseur de colorant d'oxydation additionnel différent des composés de formule (I), de manière encore plus préférée la composition selon l'invention contient au moins un coupleur.

Parmi les coupleurs d'oxydation, on peut notamment citer les méta-phénylènediamines, les méta-aminophénols, les méta-diphénols, les coupleurs naphtaléniques et les coupleurs hétérocycliques ainsi que leurs sels d'addition.

A titre d'exemple, on peut citer le 2-méthyl 5-aminophénol, le 5-N-(β-hydroxyéthyl)amino 2-méthyl phénol, le 6-chloro-2-méthyl-5-aminophénol, le 3-amino phénol, le 1,3-dihydroxy benzène (ou résorcinol), le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxybenzène, le 1,3-diamino benzène, le 1,3-bis-(2,4-diaminophénoxy) propane, la 3-uréido aniline, le 3-uréido 1-diméthylamino benzène, le sésamol, le 1-β-hydroxyéthylamino-3,4-méthylènedioxybenzène, l'α-naphtol, le 2 méthyl-1-naphtol, le 6-hydroxy indole, le 4-hydroxy indole, le 4-hydroxy N-méthyl indole, la 2-amino-3-hydroxy pyridine, la 6-hydroxy benzomorpholine, la 3,5-diamino-2,6-diméthoxypyridine, le 1-N-(β-hydroxyéthyl)amino-3,4-méthylène dioxybenzène, le 2,6-bis-(β-hydroxyéthylamino)toluène et leurs sels d'addition.

Généralement la concentration du ou des coupleurs d'oxydation est comprise entre 0,0001 et 20%, de préférence entre 0,005 à 6% en poids par rapport au poids total de la composition.

Les bases d'oxydation additionnelles différentes des composés de formule (I) peuvent notamment être choisies parmi les para-phénylènediamines, les bis-phénylalkylènediamines, les para-aminophénols, les ortho-aminophénols et les bases hétérocycliques et leurs sels d'addition.

Parmi les para-phénylènediamines, on peut plus particulièrement citer à titre d'exemple, la para-phénylènediamine, la para-toluènediamine, la 2-chloro para-phénylènediamine, la 2,3-diméthyl para-phénylènediamine, la 2,6-diméthyl para-phénylènediamine, la 2,6-diéthyl para-phénylènediamine, la 2,5-diméthyl para-phénylènediamine, la N,N-diméthyl para-phénylènediamine, la N,N-diéthyl para-phénylènediamine, la N,N-dipropyl para-phénylènediamine, la 4-amino N,N-diéthyl 3-méthyl aniline, la N,N-bis-(β-hydroxyéthyl) para-phénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl para-phénylènediamine, la 2-fluoro para-phénylènediamine, la 2-isopropyl para-phénylènediamine, la N-(β-hydroxypropyl) para-phénylènediamine, la 2-hydroxyméthyl para-phénylènediamine, la N,N-diméthyl 3-méthyl para-phénylènediamine, la N,N-(éthyl, β-hydroxyéthyl) para-phénylènediamine, la N-(β,γ-dihydroxypropyl) para-phénylènediamine, la N-(4'-aminophényl) para-phénylènediamine, la N-phényl para-phénylènediamine, la 2-β-hydroxyéthyloxy para-phénylènediamine, la 2-β-acétylaminoéthyloxy para-phénylènediamine, la N-(β-méthoxyéthyl) para-phénylènediamine, la 4 aminophényl pyrrolidine, le 2 thiényl para-phénylène diamine, le 2-β hydroxyéthylamino 5-amino toluène, la 3-hydroxy 1-(4' aminophényl)pyrrolidine, la 6-(4-Amino-phénylamino)-hexan-1-ol et leurs sels d'addition avec un acide.

Parmi les para-phénylènediamines citées ci-dessus, la para-phénylènediamine, la para-toluènediamine, la 2-isopropyl para-phénylènediamine, la 2-β-hydroxyéthyl para-phénylènediamine, la 2-β-hydroxyéthyloxy para-phénylènediamine, la 2,6-diméthyl para-phénylènediamine, la 2,6-diéthyl para-phénylènediamine, la 2,3-diméthyl para-phénylènediamine, la N,N-bis-(β-hydroxyéthyl) para-phénylènediamine, la 2-chloro para-phénylènediamine, la 2-β-acétylaminoéthyloxy para-phénylènediamine, et leurs sels d'addition avec un acide sont particulièrement préférées.

Parmi les bis-phénylalkylènediamines, on peut citer à titre d'exemple, le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthylaminophényl) tétraméthylènediamine, la N,N'-bis-(éthyl) N,N'-bis-(4'-amino, 3'-méthylphényl) éthylènediamine, le 1,8-bis-(2,5-diamino phénoxy)-3,6-dioxaoctane, et leurs sels d'addition avec un acide.

Parmi les para-aminophénols, on peut citer à titre d'exemple, le para-aminophénol, le 4-amino 3-méthyl phénol, le 4-amino 3-fluoro phénol, le 4-amino 2-chloro phénol, le 4-amino 3-chloro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, le 4-amino-2,6 dichlorophénol, le 4-amino 6[((5'amino-2'hydroxy-3'-méthyl)phényl)méthyl]-2-méthylphénol, le bis[(5'amino-2'hydroxy)phénylméthane et leurs sels d'addition avec un acide.

Parmi les ortho-aminophénols, on peut citer à titre d'exemple, le 2-amino phénol, le 2-amino 5-méthyl phénol, le 2-amino 6-méthyl phénol, le 5-acétamido 2-amino phénol, et leurs sels d'addition avec un acide.

Parmi les bases hétérocycliques, on peut citer à titre d'exemple, les dérivés pyridiniques, les dérivés pyrimidiniques et les dérivés pyrazoliques.

Parmi les dérivés pyridiniques, on peut citer les composés décrits par exemple dans les brevets GB 1 026 978 et GB 1 153 196, comme la 2,5-diamino pyridine, la 2-(4-méthoxyphényl)amino 3-amino pyridine, la 3,4-diamino pyridine, et leurs sels d'addition avec un acide.

D'autres bases d'oxydation pyridiniques utiles dans la présente invention sont les bases d'oxydation 3-amino pyrazolo-[1,5-a]-pyridines ou leurs sels d'addition décrits par exemple dans la demande de brevet FR 2801308. A titre d'exemple, on peut citer la pyrazolo[1,5-a]pyridin-3-ylamine ; la 2-acétylamino pyrazolo-[1,5-a] pyridin-3-ylamine ; la 2-morpholin-4-yl-pyrazolo[1,5-a]pyridin-3-ylamine ; l'acide 3-amino-pyrazolo[1,5-a]pyridin-2-carboxylique ; la 2-méthoxy-pyrazolo[1,5-a]pyridine-3-ylamino ; le (3-amino-pyrazolo[1,5-a]pyridine-7-yl)-méthanol ; le 2-(3-amino-pyrazolo[1,5-a]pyridine-5-yl)-éthanol ; le 2-(3-amino-pyrazolo[1,5-a]pyridine-7-yl)-éthanol ; le (3-amino-pyrazolo[1,5-a]pyridine-2-yl)-méthanol ; la 3,6-diamino-pyrazolo[1,5-a]pyridine ; la 3,4-diamino-pyrazolo[1,5-a]pyridine ; la pyrazolo[1,5-a]pyridine-3,7-diamine ; la 7-morpholin-4-yl-pyrazolo[1,5-a]pyridin-3-ylamine ; la pyrazolo[1,5-a]pyridine-3,5-diamine ; la 5-morpholin-4-yl-pyrazolo[1,5-a]pyridin-3-ylamine ; le 2-[(3-amino-pyrazolo[1,5-a]pyridin-5-yl)-(2-hydroxyéthyl)-amino]-éthanol ; le 2-[(3-amino-pyrazolo[1,5-a]pyridin-7-yl)-(2-hydroxyéthyl)-amino]-éthanol ; la 3-amino-pyrazolo[1,5-a]pyridine-5-ol ; 3-amino-pyrazolo[1,5-a]pyridine-4-ol ; la 3-amino-pyrazolo[1,5-a]pyridine-6-ol ; la 3-amino-pyrazolo[1,5-a]pyridine-7-ol ;
ainsi que leurs sels d'addition avec un acide.

Parmi les dérivés pyrimidiniques, on peut citer les composés décrits par exemple dans les brevets DE 2359399 ; JP 88-169571 ; JP 05-63124 ; EP 0770375 ou demande de brevet WO 96/15765 comme la 2,4,5,6-tétra-aminopyrimidine, la 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine et leurs sels d'addition et leurs formes tautomères, lorsqu'il existe un équilibre tautomérique.

Parmi les dérivés pyrazoliques, on peut citer les composés décrits dans les brevets DE 3843892, DE 4133957 et demandes de brevet WO 94/08969, WO 94/08970, FR-A-2 733 749 et DE 195 43 988 comme le 4,5-diamino 1-méthyl pyrazole, le 4,5-diamino 1-(β-hydroxyéthyl) pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition.

Généralement la concentration de la ou les bases d'oxydation additionnelle(s) est comprise entre 0,0001 et 20%, de préférence entre 0,005 à 6% en poids par rapport au poids total de la composition.

D'une manière générale, les sels d'addition avec un acide utilisables pour les bases d'oxydation et les coupleurs sont notamment choisis parmi les chlorhydrates, les bromhydrates, les sulfates, les citrates, les succinates, les tartrates, les lactates, les tosylates, les benzènesulfonates, les phosphates et les acétates.

La composition tinctoriale conforme à l'invention peut en outre contenir un ou plusieurs colorants directs pouvant notamment être choisis parmi les colorants nitrés de la série benzénique, neutres, acides ou cationiques, les colorants directs azoïques neutres, acides ou cationiques, les colorants directs quinoniques et en particulier anthraquinoniques neutres, acides ou cationiques, les colorants directs aziniques, les colorants directs méthiniques, azométhiniques, triarylméthaniques, indoaminiques et les colorants directs naturels. De préférence, la composition selon l'invention comprend au moins un colorant choisi parmi les colorants directs cationiques et les colorants directs naturels.

Parmi les colorants directs cationiques utilisables selon l'invention on peut citer les colorants directs azoïques cationiques décrits dans les demandes de brevets WO 95/15144, WO-95/01772 et EP-714954.

Parmi ces composés on peut tout particulièrement citer les colorants suivants :
- chlorure de 1,3-diméthyl-2-[[4-(diméthylamino)phényl]azo]-1H-Imidazolium,
- chlorure de 1,3-diméthyl-2-[(4-aminophényl)azo]-1H-Imidazolium,
- méthylsulfate de 1-méthyl-4-[(méthylphénylhydrazono) méthyl]-pyridinium.

Parmi les colorants directs naturels utilisables selon l'invention, on peut citer la lawsone, la juglone, l'alizarine, la purpurine, l'acide carminique, l'acide kermésique, la purpurogalline, le protocatéchaldéhyde, l'indigo, l'isatine, la curcumine, la spinulosine, l'apigénidine. On peut également utiliser les extraits ou décoctions contenant ces colorants naturels et notamment les cataplasmes ou extraits à base de henné.

Le ou les colorants directs représentent de préférence de 0,001 à 20% en poids environ du poids total de la composition prête à l'emploi et encore plus préférentiellement de 0,005 à 10% en poids environ.

Bien entendu, l'homme de l'art veillera à choisir le ou les adjuvants, précurseurs de colorants d'oxydations additionnels, colorants directs de manière telle que les propriétés avantageuses attachées intrinsèquement à la composition de teinture d'oxydation conforme à l'invention ne soient pas, ou substantiellement pas, altérées par la ou les adjonctions envisagées.

Le pH de la composition tinctoriale conforme à l'invention est généralement compris entre 3 et 12 environ, et de préférence entre 5 et 11 environ. Il peut être ajusté à la valeur désirée au moyen d'agents acidifiants ou alcalinisants habituellement utilisés en teinture des fibres kératiniques ou bien encore à l'aide de systèmes tampons classiques.

Parmi les agents acidifiants, on peut citer, à titre d'exemple, les acides minéraux ou organiques autres que les diacides carboxyliques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule : 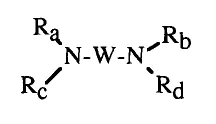 dans laquelle W est un reste propylène éventuellement substitué par un groupement hydroxyle ou un radical alkyle en C₁-C₄ ; Rₐ, R_{b}, R_{c} et R_{d}, identiques ou différents, représentent un atome d'hydrogène, un radical alkyle en C₁-C₄ ou hydroxyalkyle en C₁-C₄.

La composition cosmétique selon l'invention peut se présenter sous des formes diverses, telles que sous forme de liquides, de crèmes, de gels, ou sous toute autre forme appropriée pour réaliser une teinture des fibres kératiniques, et notamment des cheveux humains.

La présente demande concerne un procédé dans lequel on applique sur les fibres kératiniques la composition selon la présente invention telle que définie précédemment pendant une durée suffisante pour développer la coloration désirée en présence d'un agent oxydant, l'agent oxydant étant appliqué avant, simultanément ou après la composition. La couleur peut être révélée à pH acide, neutre ou alcalin et l'agent oxydant peut être ajouté à la composition de l'invention juste au moment de l'emploi ou il peut être mis en oeuvre à partir d'une composition oxydante le contenant, appliquée simultanément ou séquentiellement à la composition de l'invention.

Selon un mode de réalisation particulier, la composition selon la présente invention est mélangée, de préférence au moment de l'emploi, à une composition contenant, dans un milieu approprié pour la teinture, au moins un agent oxydant, cet agent oxydant étant présent en une quantité suffisante pour développer une coloration : selon ce mode de réalisation particulier, on dispose d'une composition prête à l'emploi qui est un mélange d'une composition selon l'invention avec au moins un agent oxydant de préférence choisi parmi le peroxyde d'hydrogène, le peroxyde d'urée, les bromates de métaux alcalins, les persels, les peracides et les enzymes oxydases. Le mélange obtenu, sous la forme d'une composition prête à l'emploi, est ensuite appliqué sur les fibres kératiniques pendant une durée suffisante pour développer la coloration désirée. Après un temps de pose de 3 à 50 minutes environ, de préférence 5 à 30 minutes environ, les fibres kératiniques sont rincées, lavées au shampooing, rincées à nouveau puis séchées.

Les agents oxydants classiquement utilisés pour la teinture d'oxydation des fibres kératiniques sont par exemple le peroxyde d'hydrogène, le peroxyde d'urée, les bromates de méta-ux alcalins, les persels tels que les perborates et persulfates, les peracides et les enzymes oxydases parmi lesquelles on peut citer les peroxydases, les oxydo-réductases à 2 électrons telles que les uricases et les oxygénases à 4 électrons comme les laccases. Le peroxyde d'hydrogène est particulièrement préféré.

La composition oxydante peut également renfermer divers adjuvants utilisés classiquement dans les compositions pour la teinture des cheveux et tels que définis précédemment.

Le pH de la composition oxydante renfermant l'agent oxydant est tel qu'après mélange avec la composition tinctoriale, le pH de la composition résultante appliquée sur les fibres kératiniques varie de préférence entre 3 et 12 environ, et encore plus préférentiellement entre 5 et 11. Il peut être ajusté à la valeur désirée au moyen d'agents acidifiants ou alcalinisants habituellement utilisés en teinture des fibres kératiniques et tels que définis précédemment.

La composition prête à l'emploi qui est finalement appliquée sur les fibres kératiniques peut se présenter sous des formes diverses, telles que sous forme de liquides, de crèmes, de gels ou sous toute autre forme appropriée pour réaliser une teinture des fibres kératiniques, et notamment des cheveux humains.

L'invention a aussi pour objet un dispositif à plusieurs compartiments ou "kit" de teinture dans lequel un premier compartiment renferme la composition tinctoriale définie ci-dessus et un deuxième compartiment renferme une composition oxydante. Ce dispositif peut être équipé d'un moyen permettant de délivrer sur les cheveux le mélange souhaité, tel que les dispositifs décrits dans le brevet FR-2 586 913 au nom de la demanderesse.

A partir de ce dispositif, il est possible de teindre les fibres kératiniques à partir d'un procédé qui comprend le mélange d'une composition tinctoriale conforme à l'invention avec un agent oxydant tel que défini précédemment, et l'application du mélange obtenu sur les fibres kératiniques pendant un temps suffisant pour développer la coloration désirée.

Les exemples qui suivent servent à illustrer l'invention sans toutefois présenter un caractère limitatif.

### EXEMPLES

### Exemple 1 : Synthèse du N-1-(2-amino-éthyl)-2-méthoxyméthyl-benzène-1,4-diamine, dichlorhydrate (4)

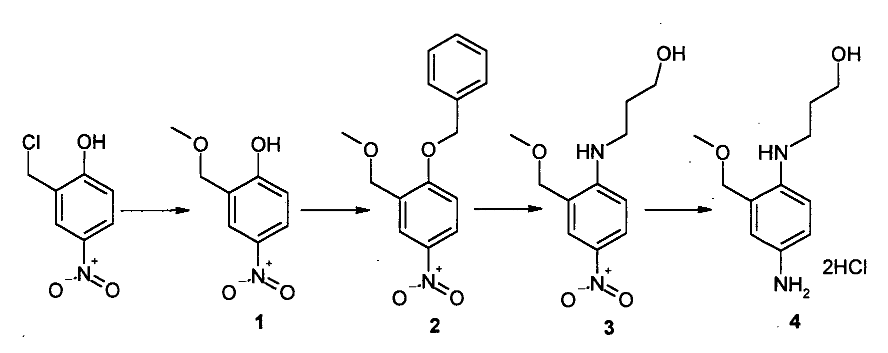

### Etape 1 : Préparation du 2-Méthoxyméthyl-4-nitro-phénol (1)

On mélange 18,7g (0,1 mole) de chlorométhyl-2-nitro-4-phénol et 50 ml de méthanol pur et on porte au reflux pendant 2 heures 30. On ajoute 37 ml de méthylate de sodium (0,2 mole, solution 5,4N dans MeOH) et on laisse encore 30 min de reflux. On refroidit dans un bain de glace, on dilue avec 350 ml d'eau et on filtre l'insoluble formé. Le filtrat est acidifié à l'acide acétique (10 ml). Le produit cristallisé est essoré, ré-empâté à l'eau glacée puis séché sur P₂O₅ à 40°C. 11,4g de produits bruts ont été isolés puis recristallisés dans 15 ml d'acétate d'isopropyle pour donner 7,5 g de produit attendu.

### Etape 2 : Préparation du 1-Benzyloxy-2-méthoxyméthyl-4-nitro-benzène (2)

18,3 g de 2-Méthoxyméthyl-4-nitrophénol (1) (0,1 mole), 8,3g (0,06 mole) de carbonate de potassium et 40 ml de diméthylformamide sont chauffés au bain-marie bouillant et on coule en 10 min 12,1 ml (0,105 mole) de chlorure de benzyle. Après 1h de chauffage supplémentaire au bain-marie bouillant, verser sur 150 g de glace. Les cristaux bruns-jaunes formés sont filtrés, ré-empâtés à l'eau glacée et lavé avec de l'éther de pétrole. Le produit humide est recristallisé dans 75 ml d'isopropanol et donne après séchage 21g de produit attendu avec un point de fusion de 108°C. Les résultats d'analyses élémentaires sont les suivants :

| | THEORIE | TROUVE |
|---|---|---|
| C | 65.93 | 65.86 |
| H | 5.53 | 5.56 |
| N | 5.13 | 5.19 |
| O | 23.42 | 23.62 |

### Etape 3 : Préparation du 2-(2-Méthoxyméthyl-4-nitro-phénylamino)-éthanol (3)

13,6g (0,05 mole) 1-Benzyloxy-2-méthoxyméthyl-4-nitrobenzène (2) et 30 ml de 3-amino-propanol sont chauffés à 150-160°C pendant 1 heure. Verser le mélange sur glace et neutraliser l'excès de 3-amino-propanol avec de l'acide chlorhydrique 36%. L'huile formée se décante puis cristallise. Après essorage, les cristaux sont lavés à l'eau puis à l'éther de pétrole. Le séchage sous vide sur P₂O₅ à 30°C donne une huile orange (7,2 g) qui cristallise à 20°C.

### Etape 4 : Préparation du 2-(4-amino-2-méthoxyméthyl-phénylamino-éthanol, dichlorhydrate (4)

Le mélange de zinc en poudre (14g), le chlorure d'ammonium (0,56g), l'eau (2,8 ml) et l'éthanol 96% (28 ml) est porté au reflux au bain-marie bouillant. Le dérivé nitré 2-(2-Méthoxyméthyl-4-nitro-phénylamino)-éthanol (3) (6,8g, 0,028 mole) est ajouté petit à petit et le chauffage est maintenu jusqu'à décoloration du milieu réactionnel. On filtre bouillant et le filtrat est récupéré sur 12 ml d'éthanol chlorhydrique 6N. Après évaporation à sec, le produit brut est repris dans 10 ml d'éthanol absolu. Les cristaux formés sont essorés et séchés sous vide et sur P₂O₅/KOH pour donner 5,6 g de produit dichlorhydraté.

Les spectres RMN du proton et de masse sont conformes à la structure attendue du produit.

### Exemple 2: synthèse du 2-[2-(4-Amino-2-méthyl-phénylamino)-éthylamino]-éthanol, dichlorhydrate (6)

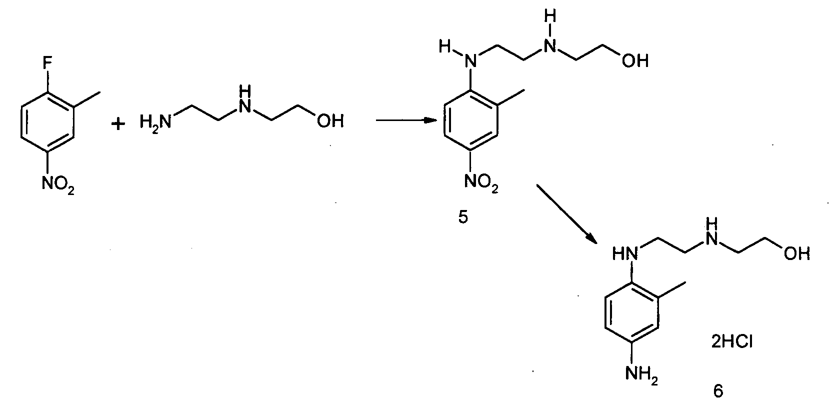

### Etape 1 : synthèse du 2-[2-(4-nitro-2-méthyl-phénylamino)-éthylamino]-éthanol (5)

A une solution de 20 ml de N-méthyl-pyrrolidinone, on ajoute 2 g de 4-fluoro-3-méthyl- nitrobenzène, 1,61 g de 2-(2-aminoéthylamino)éthanol et 2,14 g de K₂CO₃. Le milieu réactionnel est chauffé à 60°C pendant 7 heures, puis, après refroidissement à la température ambiante, est versé dans un mélange eau + glace. Le précipité jaune formé est filtré, réempaté dans l'eau puis séché sur P₂O₅. On obtient 2,4 g de 2-[2-(4-nitro-2-méthyl-phénylamino)-éthylamino]-éthanol (5).

### Etape 2 : Synthèse du 2-[2-(4-Amino-2-méthyl-phénylamino)-éthylamino]-éthanol, dichlorhydrate (6) :

Le 2-[2-(4-nitro-2-méthyl-phénylamino)-éthylamino]-éthanol (5) précédemment obtenu est réduit avec un mélange zinc / chlorure d'ammonium / eau / éthanol bouillant. L'amine correspondant est isolée sous forme de dichlorhydrate.

Les spectres RMN du proton et de masse sont conformes à la structure attendue du produit.

### Exemple 3 : synthèse du 2-[2-(4-Amino-3-méthyl-phénylamino)-éthylamino]-éthanol, dichlorhydrate (8)

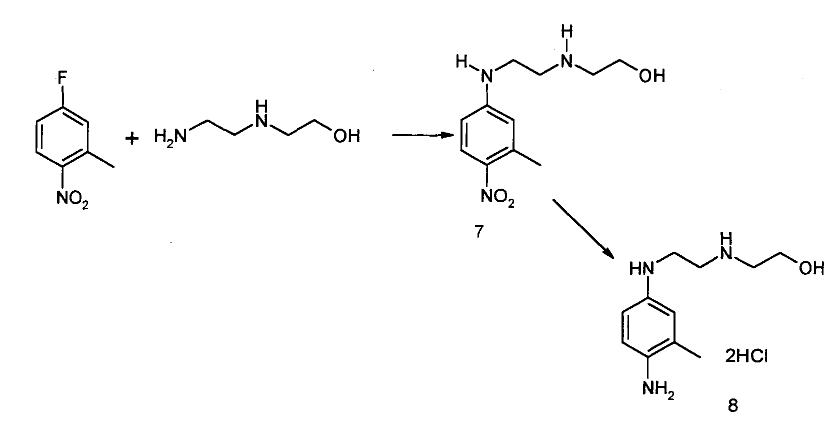

### Etape 1 : Synthèse du 2-[2-(4-nitro-3-méthyl-phénylamino)-éthylamino] éthanol (7)

A une solution de 20 ml de N-méthyl-pyrrolidinone, on ajoute 2 g de 4-fluoro-2-méthyl-nitrobenzène, 1,61 g de 2-(2-aminoéthylamino)éthanol et 2,14 g de K₂CO₃. Le milieu réactionnel est chauffé à 60°C pendant 7 heures, puis, après refroidissement à température ambiante, est versé dans un mélange eau + glace. Le précipité jaune formé est filtré, réempaté dans l'eau puis séché sur P₂O₅. On obtient 1,3 g de 2-[2-(4-nitro-3-méthyl-phénylamino)-éthylamino]-éthanol (7).

### Etape 2 : Synthèse du 2-[2-(4-Amino-3-méthyl-phénylamino)-éthylamino]-éthanol, dichlorhydrate (8) :

Le 2-[2-(4-nitro-3-méthyl-phénylamino)-éthylamino]-éthanol (7) précédemment obtenu est réduit avec un mélange zinc / chlorure d'ammonium / eau / éthanol bouillant. L'amine correspondante est isolée sous forme de dichlorhydrate.

Les spectres RMN du proton et de masse sont conformes à la structure attendue du produit.

### Exemple 4: Synthèse du 6-(4-Amino-3-méthyl-phénylamino)-hexan-1-ol, dichlorhydrate (10)

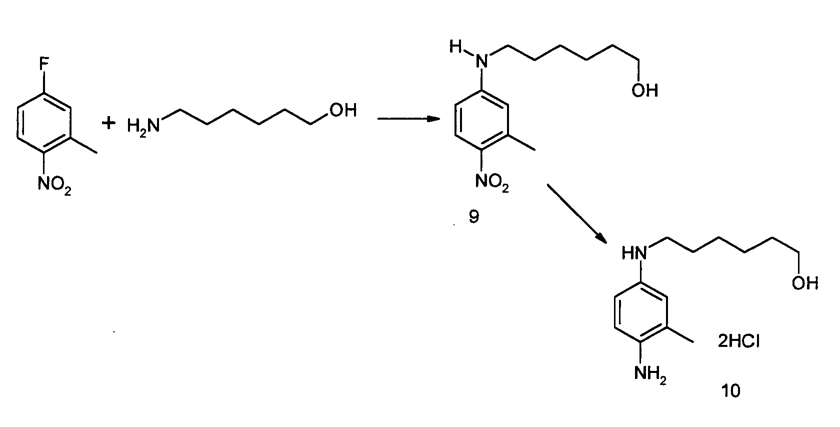

### Etape 1 : Synthèse du 6-(4-nitro-3-méthyl-phénylamino)-hexan-1-ol (9)

A une solution de 100 ml de N-méthyl-pyrrolidinone, on ajoute 10 g de 4-fluoro-2-méthyl-nitrobenzène, 9,97 g de 6-amino-1-capronol et 11,76 g de K₂CO₃. Le milieu réactionnel est chauffé à 70°C pendant 8 heures, puis, après refroidissement à température ambiante, est versé dans de l'eau. La phase aqueuse est extraite avec de l'acétate d'éthyle puis la phase organique est séchée sur MgSO₄, est évaporée à sec pour obtenir une huile. Cette huile est purifiée par chromatographie sur colonne de silice (éluant : heptane / acétate d'éthyle). On obtient 17,8 g de 6-(4-nitro-3-méthyl-phénylamino)-hexan-1-ol (9) sous forme d'un solide jaune.

### Etape 2 : synthèse du 6-(4-Amino-3-méthyl-phénylamino)-hexan-1-ol, dichlorhydrate (10)

Le 6-(4-nitro-3-méthyl-phénylamino)-hexan-1-ol (9) précédemment obtenu est réduit avec un mélange zinc / chlorure d'ammonium/ Eau/ Ethanol bouillant. L'amine correspondant est isolée sous forme de dichlorhydrate.

Les spectres RMN du proton et de masse sont conformes à la structure attendue du produit.

### Exemple 5: Synthèse du 6-(4-Amino-2-méthyl-phénylamino)-hexan-1-ol, dichlorhydrate (12)

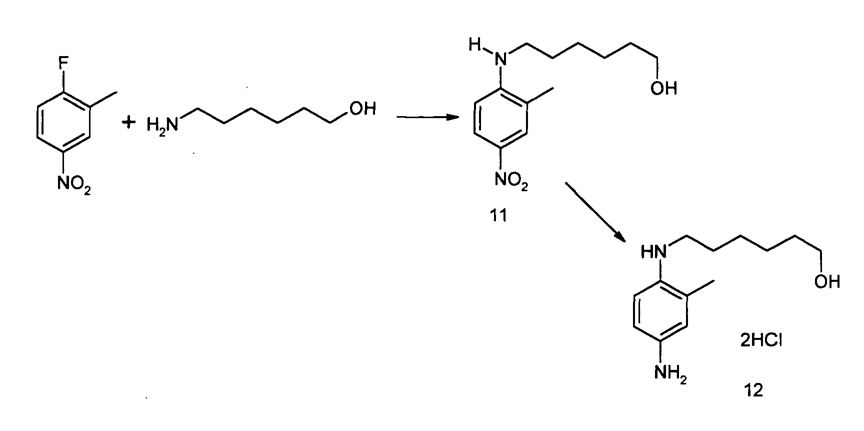

### Etape 1 : synthèse du 6-(4-nitro-2-méthyl-phénylamino)-hexan-1-ol (11)

A une solution de 50 ml de N-méthyl-pyrrolidinone, on ajoute 5 g de 4-fluoro-3-méthyl-nitrobenzène, 4,53 g de 6-amino-1-capronol et 5,35 g de K₂CO₃. Le milieu réactionnel est chauffé à 70°C pendant 7h, puis, après refroidissement à température ambiante, est versé dans un mélange eau + glace. Le précipité jaune formé est filtré, ré-empaté dans l'eau puis séché sur P₂O₅. On obtient 5,9 g de 6-(4-nitro-2-méthyl-phénylamino)-hexan-1-ol (11).

### Etape 2 : Synthèse du 6-(4-Amino-2-méthyl-phénylamino)-hexan-1-ol, dichlorhydrate 12)

Le 6-(4-nitro-2-méthyl-phénylamino)-hexan-1-ol (11) précédemment obtenu est réduit avec un mélange zinc / chlorure d'ammonium/ Eau/ Ethanol bouillant. L'amine correspondante est isolée sous forme de dichlorhydrate.

Les spectres RMN du proton et de masse sont conformes à la structure attendue du produit.

### Exemple 6 : Synthèse du 2-[2-(4-Amino-2-methyl-phenylamino)-ethoxy]-ethanol, dichlorhydrate (14)

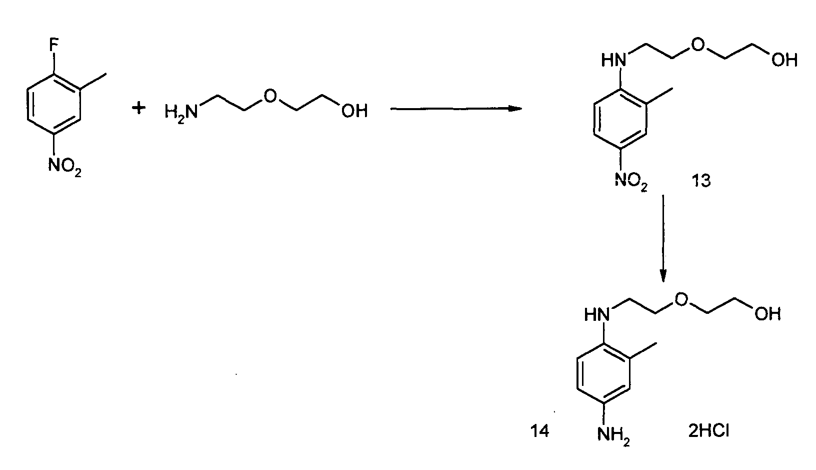

### Synthèse du 2-[2-(4-nitro-2-methyl-phenylamino)-ethoxy]-ethanol (13)

A une solution de 20 ml de N-méthyl-pyrrolidinone, 1,63 g de 2-aminoéthoxyéthanol et 2,14 g de K₂CO₃ , on ajoute 2 g de 2-fluoro-5-nitrotoluène. Le milieu réactionnel est chauffé à 60°C pendant 12 heures, puis, après refroidissement à la température ambiante, est versé dans un mélange eau + glace. Le milieu résultant est extrait avec de l'acétate d'éthyle puis la phase organique est concentré sous vide. On obtient 2,2 g de 2-[2-(4-nitro-2-methyl-phenylamino)-ethoxy]-ethanol (13)

### Synthèse du 2-[2-(4-Amino-2-methyl-phenylamino)-ethoxy]-ethanol, dichlorhydrate (14)

Le 2-[2-(4-nitro-2-methyl-phenylamino)-ethoxy]-ethanol (13) précédemment obtenu est réduit avec un mélange zinc / chlorure d'ammonium/ Eau/ Ethanol bouillant. L'amine correspondante est isolée sous forme de dichlorhydrate.

Les spectres RMN du proton et de masse sont conformes à la structure attendue du produit.

### Exemple 7 : Synthèse du 2-(2-(4-Amino-3-methyl-phenylamino)-ethoxy]-ethanol, dichlorhydrate (16)

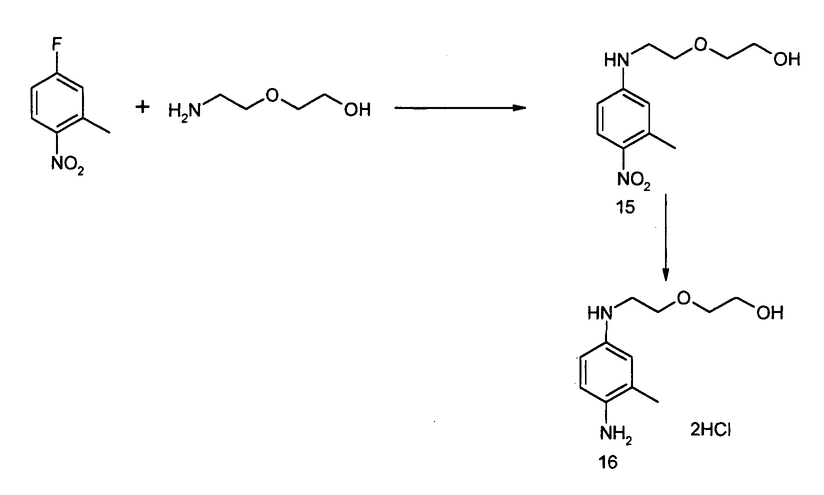

### Synthèse du 2-[2-(4-nitro-3-methyl-phenylamino)-ethoxy]-ethanol (15)

A une solution de 20 ml de N-méthyl-pyrrolidinone, 1,63 g de 2-aminoéthoxyéthanol et 2,14 g de K₂CO₃ , on ajoute 2 g de 5-fluoro-2-nitrotoluène. Le milieu réactionnel est chauffé à 60°C pendant 12 heures, puis, après refroidissement à la température ambiante, est versé dans un mélange eau + glace. Le précipité jaune formé est filtré, ré-empaté dans l'eau puis séché sur P₂O₅. On obtient 1,9 g de 2-[2-(4-nitro-3-methyl-phenylamino)-ethoxy]-ethanol (15) après purification sur colonne de silice.

### Synthèse du 2-[2-(4-Amino-3-methyl-phenylamino)-ethoxy]-ethanol, dichlorhydrate (16)

Le 2-[2-(4-nitro-2-methyl-phenylamino)-ethoxy]-ethanol (15) précédemment obtenu est réduit avec un mélange zinc / chlorure d'ammonium/ Eau/ Ethanol bouillant. L'amine correspondante est isolée sous forme de dichlorhydrate.

Les spectres RMN du proton et de masse sont conformes à la structure attendue du produit.

### Exemple 8 : Synthèse du 5-(4-Amino-3-methyl-phenylamino)-pentan-1-ol, dichlorhydrate (18)

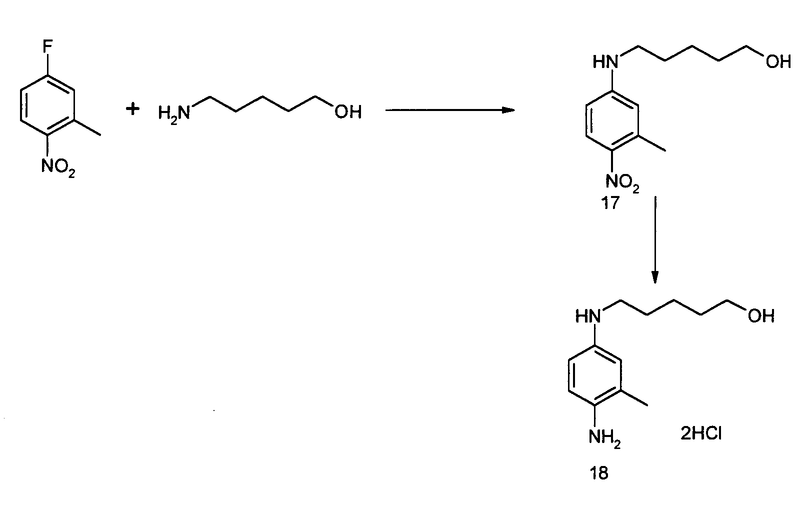

### Synthèse du 5-(4-nitro-3-methyl-phenylamino)-pentan-1-ol (17)

A une solution de 20 ml de N-méthyl-pyrrolidinone, 1,6 g de 5-amino-1-pentanol et 2,14 g de K₂CO₃ , on ajoute 2 g de 5-fluoro-2-nitrotoluène. Le milieu réactionnel est chauffé à 60°C pendant 8 heures, puis, après refroidissement à la température ambiante, est versé dans un mélange eau + glace. Le précipité jaune formé est filtré, ré-empaté dans l'eau puis séché sur P₂O₅. On obtient 2,38 g de 5-(4-nitro-3-methyl-phenylamino)-pentan-1-ol (17).

### Synthèse du 5-(4-Amino-3-methyl-phenylamino)-pentan-1-ol, dichlorhydrate (18).

Le 5-(4-nitro-3-methyl-phenylamino)-pentan-1-ol (17) précédemment obtenu est réduit avec un mélange zinc / chlorure d'ammonium/ Eau/ Ethanol bouillant. L'amine correspondante est isolée sous forme de dichlorhydrate.

Les spectres RMN du proton et de masse sont conformes à la structure attendue du produit.

### Exemple 9 : Synthèse du 5-(4-Amino-2-methyl-phenylamino)-pentan-1-ol, dichlorhydrate (20)

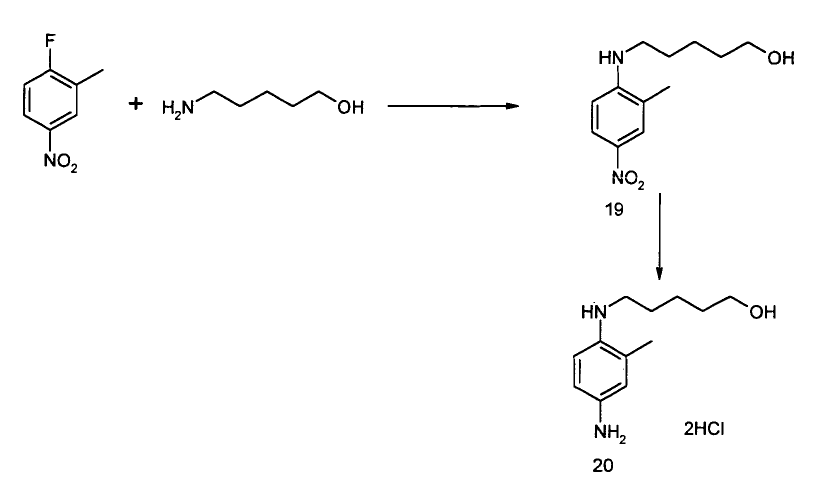

### Synthèse du 5-(4-nitro-2-methyl-phenylamino)-pentan-1-ol (19)

A une solution de 20 ml de N-méthyl-pyrrolidinone, 1,6 g de 5-amino-1-pentanol et 2,14 g de K₂CO₃, on ajoute 2 g de 2-fluoro-5-nitrotoluène. Le milieu réactionnel est chauffé à 60°C pendant 12 heures, puis, après refroidissement à la température ambiante, est versé dans un mélange eau + glace. Le précipité jaune formé est filtré, ré-empaté dans l'eau puis séché sur P₂O₅. On obtient 2,9 g de 5-(4-nitro-2-methyl-phenylamino)-pentan-1-ol (19).

### Synthèse du 5-(4-Amino-2-methyl-phenylamino)-pentan-1-ol, dichlorhydrate (20)

Le 5-(4-nitro-2-methyl-phenylamino)-pentan-1-ol (19) précédemment obtenu est réduit avec un mélange zinc / chlorure d'ammonium/ Eau/ Ethanol bouillant. L'amine correspondante est isolée sous forme de dichlorhydrate.

Les spectres RMN du proton et de masse sont conformes à la structure attendue du produit.

### Exemple 10 : Synthèse du 5-(4-Amino-2-methyl-phenylamino)-butan-1-ol, dichlorhydrate (22)

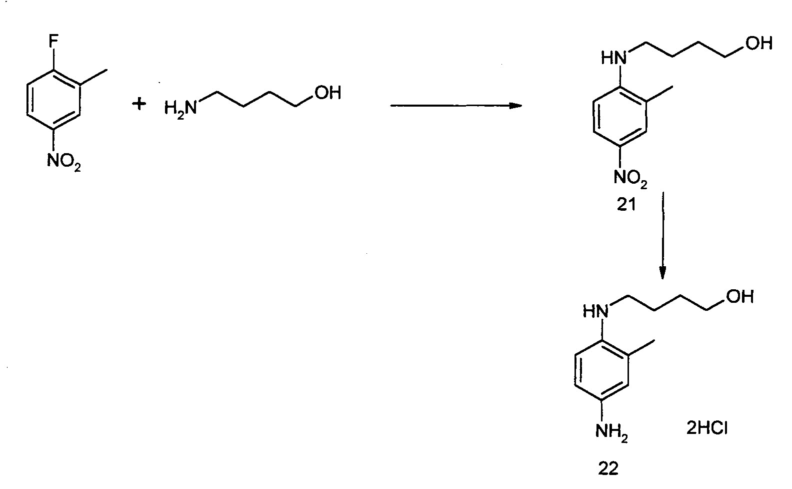

### Synthèse du 5-(4-nitro-2-methyl-phenylamino)-butan-1-ol (21)

A une solution de 20 ml de N-méthyl-pyrrolidinone, on ajoute 2 g de 2-fluoro-5-nitrotoluène, 1,38 g de 4-butanolamine et 2,14 g de K₂CO₃. Le milieu réactionnel est chauffé à 60°C pendant 9 heures, puis, après refroidissement à température ambiante, est versé dans un mélange eau + glace. Le précipité jaune formé est filtré, réempaté dans l'eau puis séché sur P₂O₅. On obtient 2,2 g de 5-(4-nitro-2-methyl-phenylamino)-butan-1-ol (21).

### Synthèse du 5-(4-Amino-2-methyl-phenylamino)-butan-1-ol, dichlorhydrate (22)

Le 5-(4-nitro-2-methyl-phenylamino)-butan-1-ol (21) précédemment obtenu est réduit avec un mélange zinc / chlorure d'ammonium/ Eau/ Ethanol bouillant. L'amine correspondante est isolée sous forme de dichlorhydrate.

Les spectres RMN du proton et de masse sont conformes à la structure attendue du produit.

### Exemple 11 : Synthèse du 5-(4-Amino-2-methyl-phenylamino)-propan-1-ol, dichlorhydrate (24)

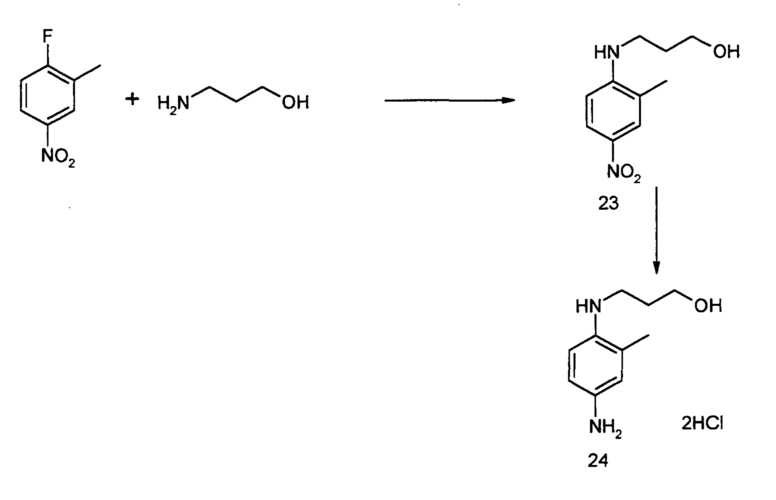

### Synthèse du 5-(4-nitro-2-methyl-phenylamino)-propan-1-ol (23)

A une solution de 20 ml de N-méthyl-pyrrolidinone, 1,16 g de N-propanolamine et 2,14 g de K₂CO₃ , on ajoute 2 g de 2-fluoro-5-nitrotoluène. Le milieu réactionnel est chauffé à 60°C pendant 12 heures, puis, après refroidissement à la température ambiante, est versé dans un mélange eau + glace. Le précipité jaune formé est filtré, ré-empaté dans l'eau puis séché sur P₂O₅. On obtient 2,15 g de 5-(4-nitro-2-methyl-phenylamino)-propan-1-ol (23).

### Synthèse du 5-(4-Amino-2-methyl-phenylamino)-propan-1-ol, dichlorhydrate (24)

Le 5-(4-nitro-2-methyl-phenylamino)-propan-1-ol (23) précédemment obtenu est réduit avec un mélange zinc / chlorure d'ammonium/ Eau/ Ethanol bouillant. L'amine correspondante est isolée sous forme de dichlorhydrate.

Les spectres RMN du proton et de masse sont conformes à la structure attendue du produit.

### Exemple 12 : Synthèse du 3-(4-Amino-2-methyl-phenylamino)-2,2-dimethyl-propan-1-ol, dichlorhydrate (26)

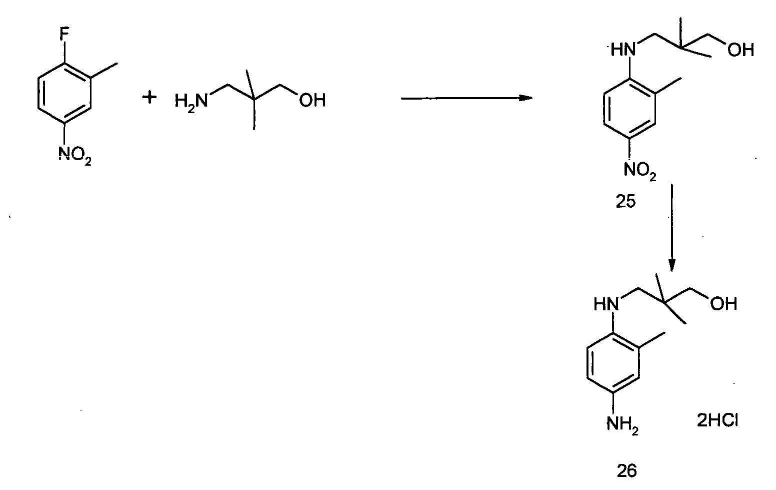

### Etape 1 : synthèse du 3-(4-nitro-2-methyl-phenylamino)-2,2-dimethyl-propan-1-ol

A une solution de 20 ml de N-méthyl-pyrrolidinone, on ajoute 2 g de 2-fluoro-5-nitrotoluène, 1,59 g de 2,2-dimethyl-3-amino-1-propanol et 2,14 g de K₂CO₃. Le milieu réactionnel est chauffé à 60°C pendant 18 heures, puis, après refroidissement à la température ambiante, est versé dans un mélange eau + glace. Le précipité jaune formé est filtré, ré-empaté dans l'eau puis séché sur P₂O₅. On obtient 1,7 g de 3-(4-nitro-2-methyl-phenylamino)-2,2-dimethyl-propan-1-ol (26)

### Etape 2 : synthèse du 3-(4-Amino-2-methyl-phenylamino)-2,2-dimethyl-propan-1-ol, dichlorhydrate

Le 3-(4-nitro-2-methyl-phenylamino)-2,2-dimethyl-propan-1-ol (26) précédemment obtenu est réduit avec un mélange zinc / chlorure d'ammonium/ Eau/ Ethanol bouillant. L'amine correspondante est isolée sous forme de dichlorhydrate.

Les spectres RMN du proton et de masse sont conformes à la structure attendue du produit.

### Exemple 13 : Synthèse du 4-(4-Amino-2-methyl-phenylamino)-butan-2-ol, dichlorhydrate (28)

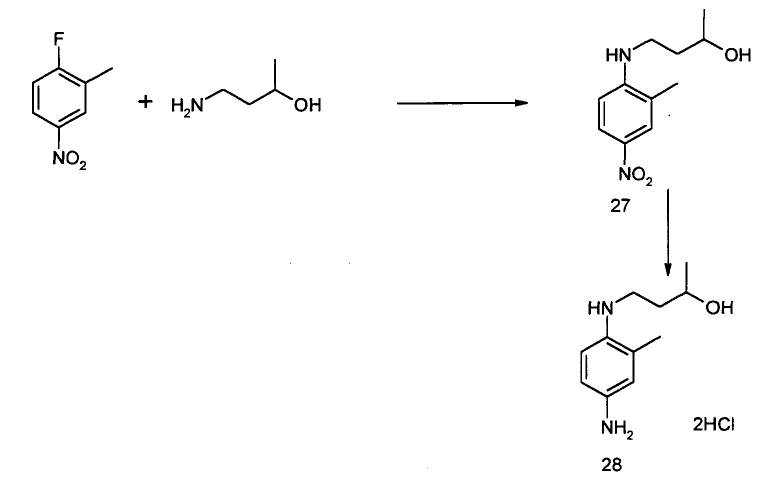

### Etape 1 : synthèse du 4-(4-nitro-2-methyl-phenylamino)-butan-2-ol (27)

A une solution de 20 ml de N-méthyl-pyrrolidinone, 1,38 g de 4-amino-2-butanol et 1,57 g de triéthylamine, on ajoute 2 g de 2-fluoro-5-nitrotoluène. Le milieu réactionnel est chauffé à 60°C pendant 12 heures, puis, après refroidissement à la température ambiante, est versé dans un mélange eau + glace. Le précipité jaune formé est filtré, ré-empaté dans l'eau puis séché sur P₂O₅. On obtient 2,38 g de 4-(4-nitro-2-methyl-phenylamino)-butan-2-ol (27).

### Etape 2 : synthèse du 4-(4-Amino-2-methyl-phenylamino)-butan-2-ol, dichlorhydrate

Le 4-(4-nitro-2-methyl-phenylamino)-butan-2-ol (27) précédemment obtenu est réduit avec un mélange zinc / chlorure d'ammonium/ Eau/ Ethanol bouillant. L'amine correspondante est isolée sous forme de dichlorhydrate.

Les spectres RMN du proton et de masse sont conformes à la structure attendue du produit.

### Exemple 14 : Synthèse du 5-(4-Amino-3-methyl-phenylamino)-butan-1-ol, dichlorhydrate (30)

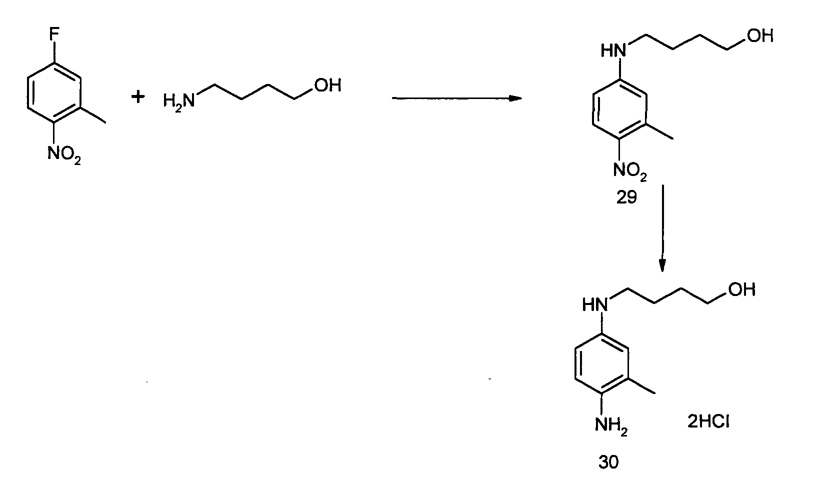

### Synthèse du 5-(4-nitro-2-methyl-phenylamino)-butan-1-ol (29)

A une solution de 20 ml de N-méthyl-pyrrolidinone, 1,38 g de 4-amino-1-butanol et 1,57 g de triéthylamine, on ajoute 2 g de 5-fluoro-2-nitrotoluène. Le milieu réactionnel est chauffé à 60°C pendant 12 heures, puis, après refroidissement à la température ambiante, est versé dans un mélange eau + glace. Le milieu résultant est extrait à l'acétate d'éthyle puis la phase organique est concentrée sous vide. On obtient 2 g de 5-(4-nitro-2-methyl-phenylamino)-butan-1-ol (29).

### Synthèse du 5-(4-Amino-2-methyl-phenylamino)-butan-1-ol, dichlorhydrate (30)

Le 5-(4-nitro-2-methyl-phenylamino)-butan-1-ol (29) précédemment obtenu est réduit avec un mélange zinc / chlorure d'ammonium/ Eau/ Ethanol bouillant. L'amine correspondante est isolée sous forme de dichlorhydrate.

Les spectres RMN du proton et de masse sont conformes à la structure attendue du produit.

### Exemple 15 : Synthèse du 4-(4-Amino-3-methyl-phenylamino)-butan-2-ol, dichlorhydrate (32)

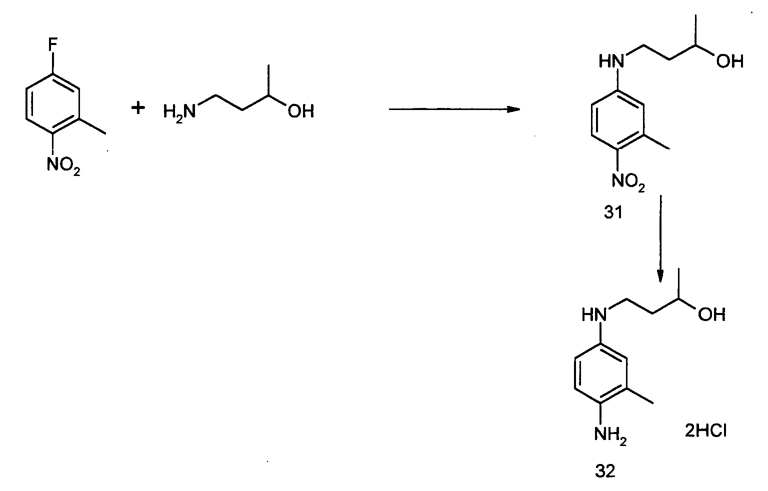

### Etape 1 : synthèse du 4-(4-nitro-3-methyl-phenylamino)-butan-2-ol (31)

A une solution de 20 ml de N-méthyl-pyrrolidinone, 1,38 g de 4-amino-2-butanol et 1,57 g de triéthylamine, on ajoute 2 g de 5-fluoro-2-nitrotoluène. Le milieu réactionnel est chauffé à 60°C pendant 10 heures, puis, après refroidissement à la température ambiante, est versé dans un mélange eau + glace. Le milieu résultant est extrait à l'acétate d'éthyle puis la phase organique est concentrée sous vide. On obtient 2,88 g de 4-(4-nitro-3-methyl-phenylamino)-butan-2-ol (31).

### Etape 2 : synthèse du 4-(4-Amino-3-methyl-phenylamino)-butan-2-ol, dichlorhydrate (32)

Le 4-(4-nitro-3-methyl-phenylamino)-butan-2-ol (31) précédemment obtenu est réduit avec un mélange zinc / chlorure d'ammonium/ Eau/ Ethanol bouillant. L'amine correspondante est isolée sous forme de dichlorhydrate.

Les spectres RMN du proton et de masse sont conformes à la structure attendue du produit.

### Exemple 16 : Synthèse du 5-(4-Amino-3-methyl-phenylamino)-propan-1-ol, dichlorhydrate (34)

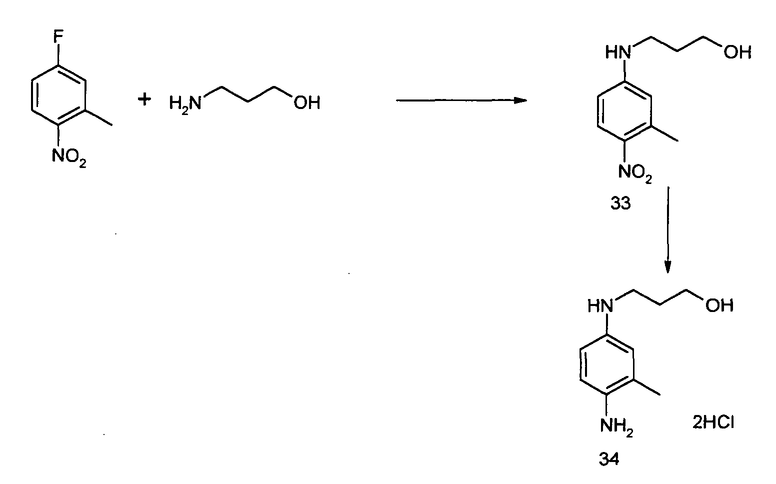

### Synthèse du 5-(4-nitro-3-methyl-phenylamino)-propan-1-ol (33)

A une solution de 20 ml de N-méthyl-pyrrolidinone, 1,16 g de N-propanolamine et 2,14 g de K₂CO₃, on ajoute 2 g de 5-fluoro-2-nitrotoluène. Le milieu réactionnel est chauffé à 60°C pendant 12 heures, puis, après refroidissement à la température ambiante, est versé dans un mélange eau + glace. Le précipité jaune formé est filtré, ré-empaté dans l'eau puis séché sur P₂O₅. On obtient 2 g de 5-(4-nitro-3-methyl-phenylamino)-propan-1-ol (33).

### Synthèse du 5-(4-Amino-3-methyl-phenylamino)-propan-1-ol, dichlorhydrate (34)

Le 5-(4-nitro-3-methyl-phenylamino)-propan-1-ol (33) précédemment obtenu est réduit avec un mélange zinc / chlorure d'ammonium/ Eau/ Ethanol bouillant. L'amine correspondante est isolée sous forme de dichlorhydrate.

Les spectres RMN du proton et de masse sont conformes à la structure attendue du produit.

### EXEMPLES DE TEINTURE

### Exemples 1 à 3 : Composition tinctoriale à partir du 2-[2-(4-Amino-2-méthyl-phénylamino)-éthylamino]-éthanol, dichlorhydrate (6)

### - Exemples 1 à 3: Teinture en milieu acide

Les compositions tinctoriales suivantes sont préparées :

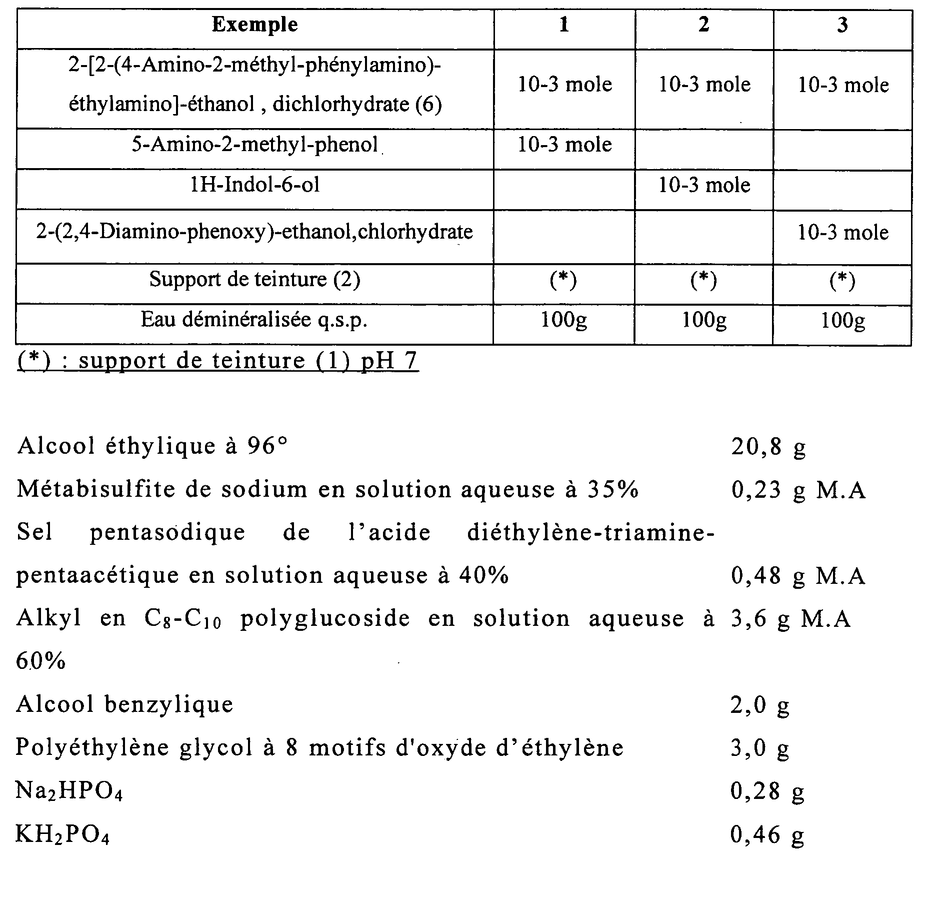

Au moment de l'emploi, chaque composition est mélangée avec un poids égal d'eau oxygénée à 20 volumes (6% en poids). On obtient un pH final de 7.

Chaque mélange obtenu est appliqué sur des mèches de cheveux gris à 90 % de blancs. Après 30 minutes de pause, les mèches sont rincées, lavées avec un shampooing standard, rincées à nouveau puis séchées.

Les nuances obtenues figurent dans le tableau ci-dessous :

| Exemple | 1 | 2 | 3 |
|---|---|---|---|
| Nuance observée | orangé | orangé | brun intense |

### Exemples 4 à 15: Composition tinctoriale à partir du 5-(4-Amino-2-methyl-phenylamino)-pentan-1-ol, dichlorhydrate (20)

### - Exemples 4 à 10 : Teinture en milieu acide

Les compositions tinctoriales suivantes sont préparées :

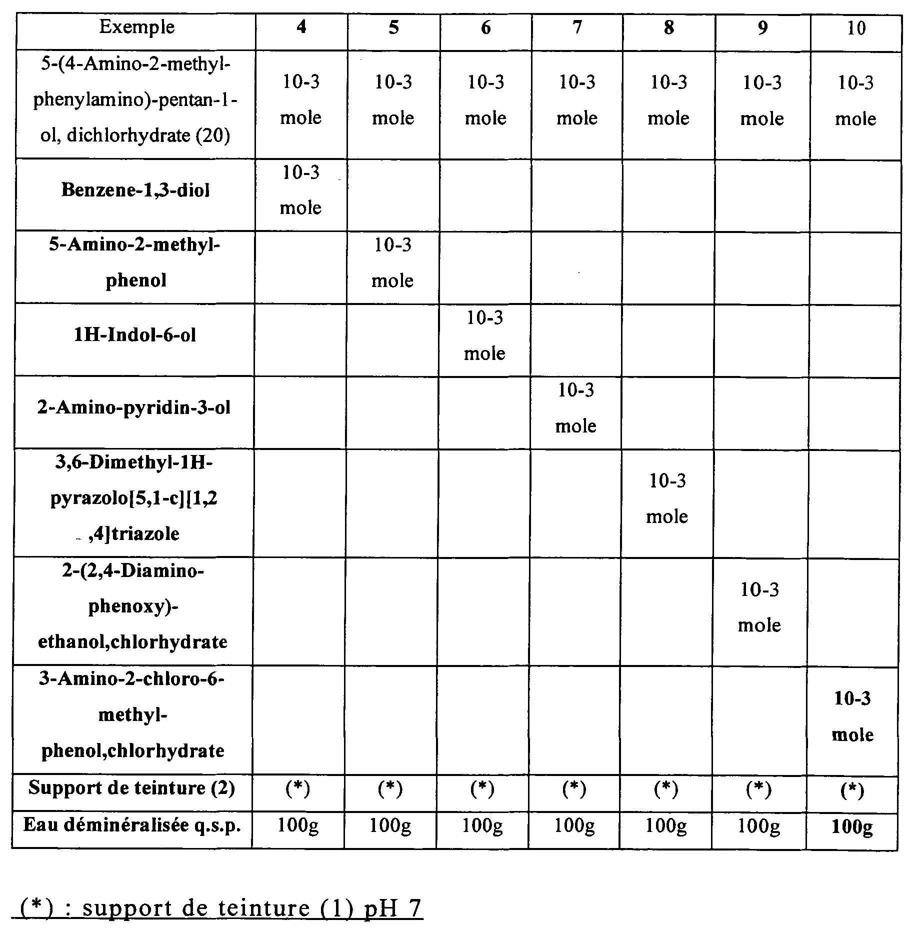

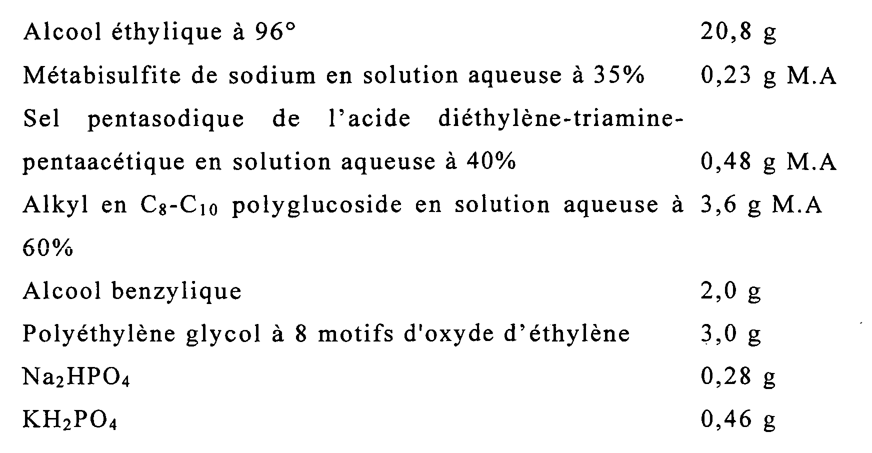

Au moment de l'emploi, chaque composition est mélangée avec un poids égal d'eau oxygénée à 20 volumes (6% en poids). On obtient un pH final de 7.

Chaque mélange obtenu est appliqué sur des mèches de cheveux gris à 90 % de blancs. Après 30 minutes de pause, les mèches sont rincées, lavées avec un shampooing standard, rincées à nouveau puis séchées.

Les nuances obtenues figurent dans le tableau ci-dessous :

| Exemple | 4 | 5 | 6 | 7 | 8 | 9 | 10 |
|---|---|---|---|---|---|---|---|
| Nuance observée | brun jaune | gris violet-bleu intense | gris intense | brun intense | rouge | bleu intense | violet-bleu intense |

### - Exemples 11 à 15 : teinture en milieu basique

Les compositions tinctoriales suivantes sont préparées :

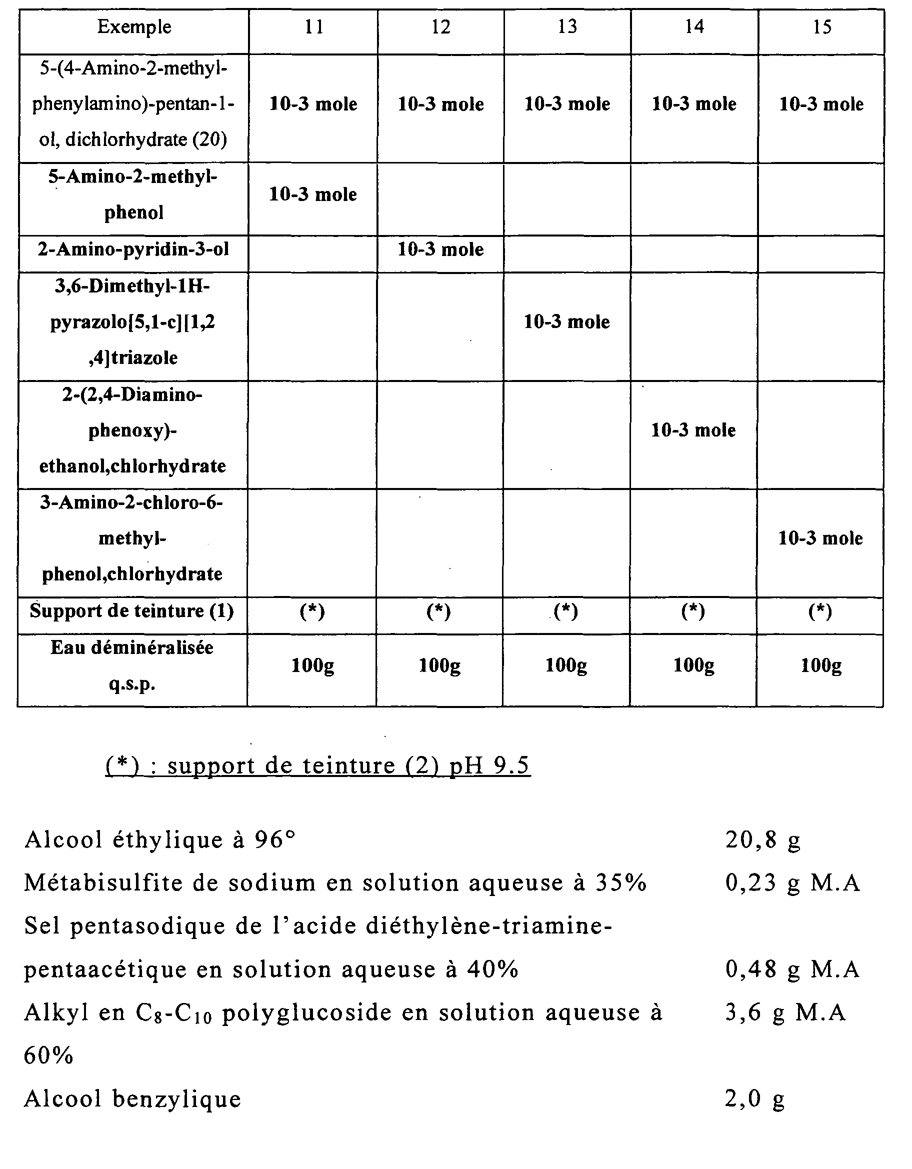

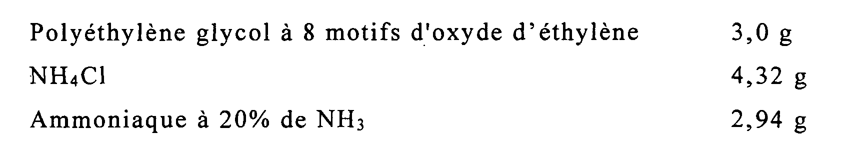

Au moment de l'emploi, chaque composition est mélangée avec un poids égal d'eau oxygénée à 20 volumes (6% en poids). On obtient un pH final de 9,5.

Chaque mélange obtenu est appliqué sur des mèches de cheveux gris à 90 % de blancs. Après 30 minutes de pause, les mèches sont rincées, lavées avec un shampooing standard, rincées à nouveau puis séchées.

Les nuances obtenues figurent dans le tableau ci-dessous :

| **Exemple** | **11** | **12** | **13** | **14** | **15** |
|---|---|---|---|---|---|
| **Nuance observée** | violet intense | brun orangé | violet-rouge chromatique | bleu intense | bleu intense |

### Exemples 16 à 26 : Composition tinctoriale à partir du 2-[2-(4-Amino-2-methyl-phenylamino)-ethoxy]-ethanol, dichlorhydrate (14)

### - Exemples 16 à 22 : Teinture en milieu acide

Les compositions tinctoriales suivantes sont préparées :

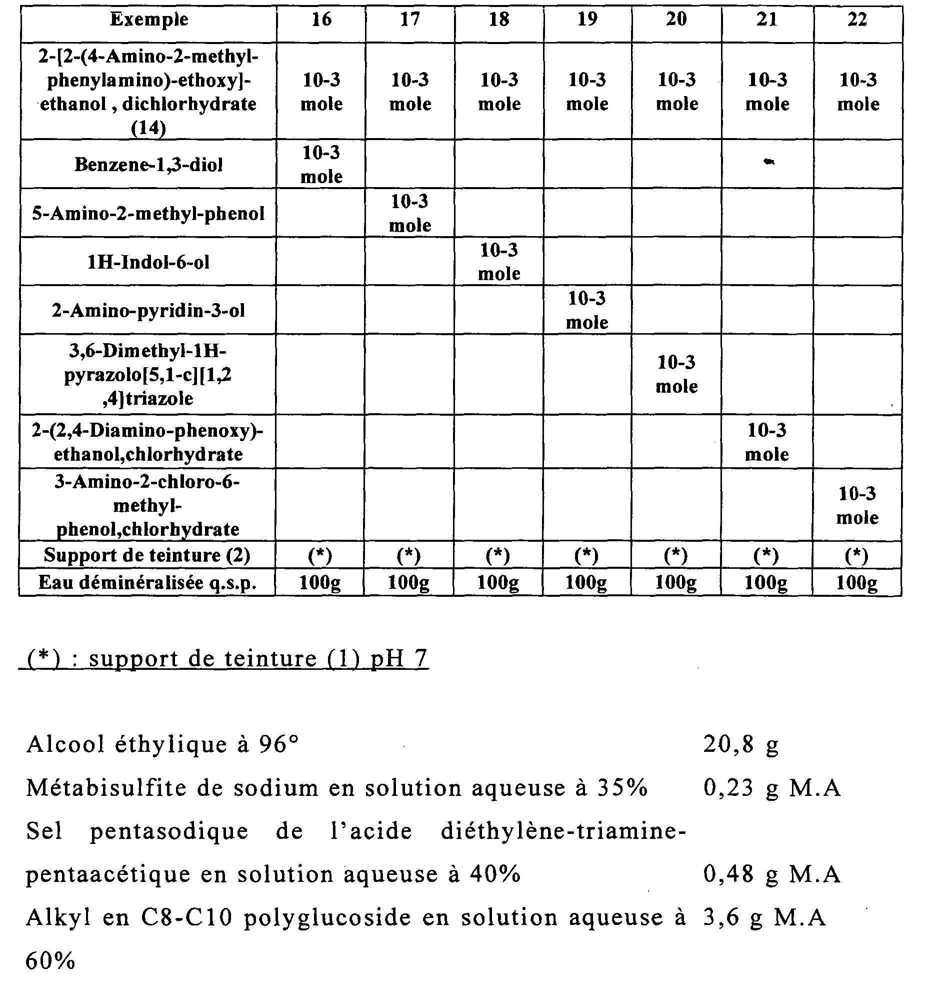

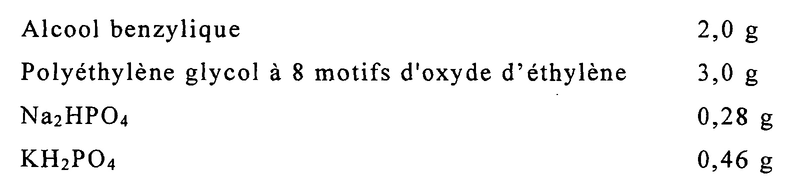

Au moment de l'emploi, chaque composition est mélangée avec un poids égal d'eau oxygénée à 20 volumes (6% en poids). On obtient un pH final de 7.

Chaque mélange obtenu est appliqué sur des mèches de cheveux gris à 90 % de blancs. Après 30 minutes de pause, les mèches sont rincées, lavées avec un shampooing standard, rincées à nouveau puis séchées.

Les nuances obtenues figurent dans le tableau ci-dessous :

| Exemple | 16 | 17 | 18 | 19 | 20 | 21 | 22 |
|---|---|---|---|---|---|---|---|
| Nuance observée | brun jaune | gris violet intense | brun orangé | brun rouge | orangé | bleu intense | violet-bleu intense |

### - Exemples 23 à 26: teinture en milieu basique

Les compositions tinctoriales suivantes sont préparées :

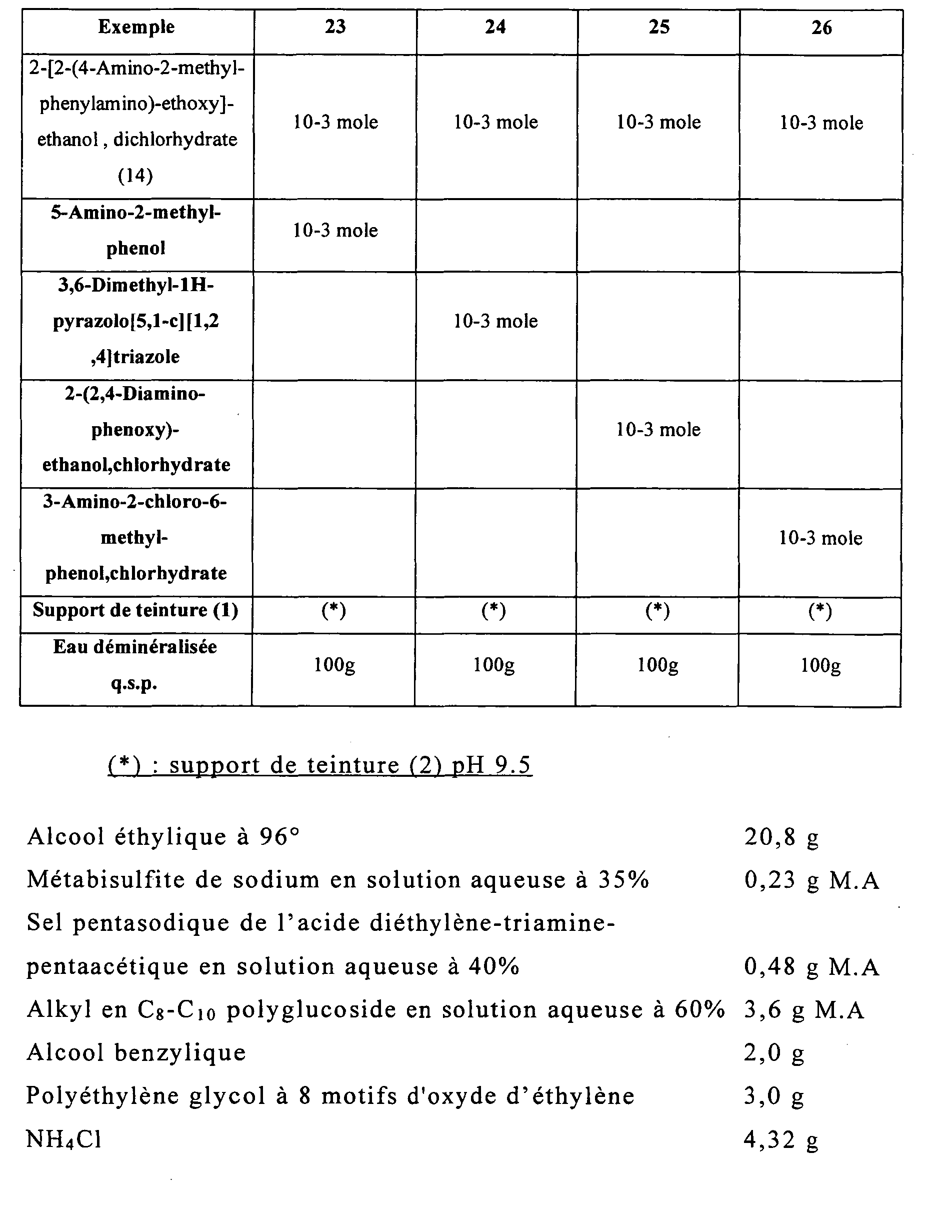

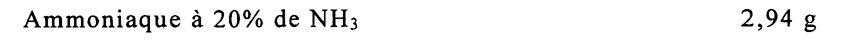

Au moment de l'emploi, chaque composition est mélangée avec un poids égal d'eau oxygénée à 20 volumes (6% en poids). On obtient un pH final de 9,5.

Chaque mélange obtenu est appliqué sur des mèches de cheveux gris à 90 % de blancs. Après 30 minutes de pause, les mèches sont rincées, lavées avec un shampooing standard, rincées à nouveau puis séchées.

Les nuances obtenues figurent dans le tableau ci-dessous :

| Exemple | 23 | 24 | 25 | 26 |
|---|---|---|---|---|
| Nuance observée | **violet- rouge** | **rouge chromatiq ue** | **bleu intense** | **violet-bleu intense** |

### Exemples 27 à 30 : Composition tinctoriale à partir du 2-[2-(4-Amino-3-méthyl-phénylamino)-éthylamino]-éthanol, dichlorhydrate (8)

### - Exemples 27 à 29 : Teinture en milieu acide

Les compositions tinctoriales suivantes sont préparées :

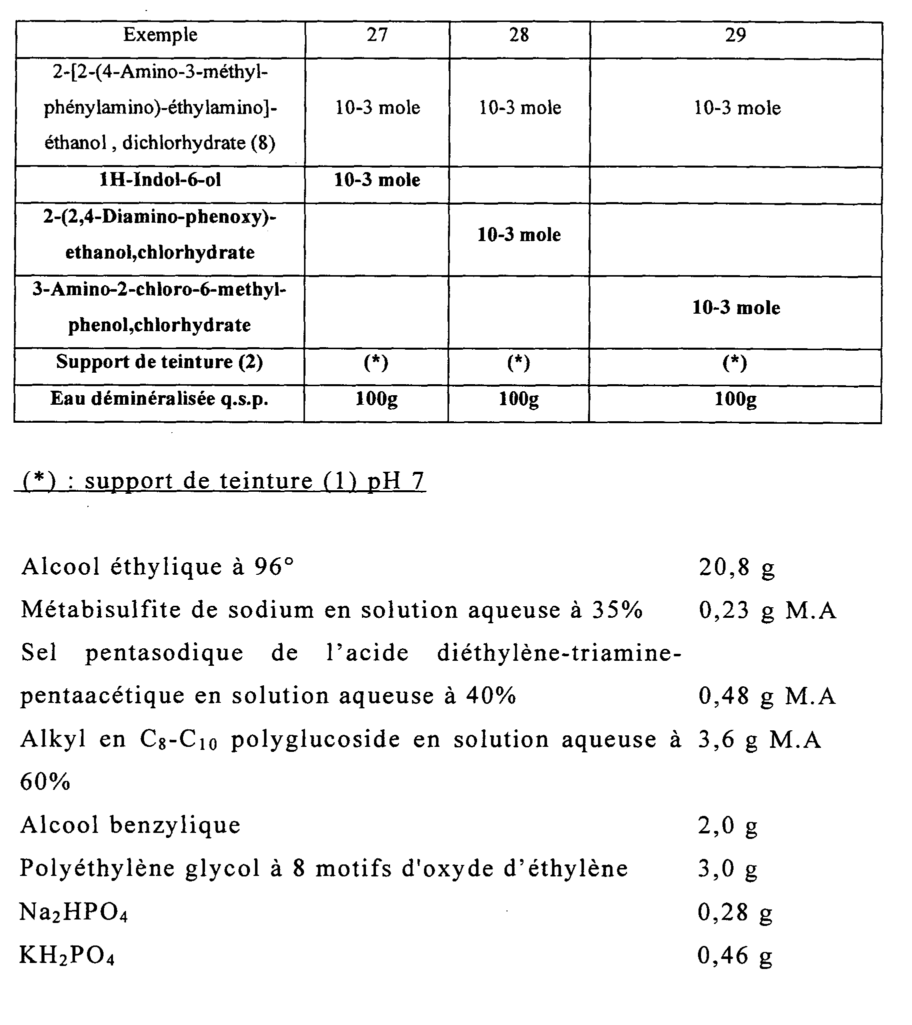

Au moment de l'emploi, chaque composition est mélangée avec un poids égal d'eau oxygénée à 20 volumes (6% en poids). On obtient un pH final de 7.

Chaque mélange obtenu est appliqué sur des mèches de cheveux gris à 90 % de blancs. Après 30 minutes de pause, les mèches sont rincées, lavées avec un shampooing standard, rincées à nouveau puis séchées.

Les nuances obtenues figurent dans le tableau ci-dessous :

| **Exemple** | **27** | **28** | **29** |
|---|---|---|---|
| **Nuance observée** | orangé | gris vert-bleu intense | brun orangé |

### - Exemples 30 : teinture en milieu basique

La composition tinctoriale suivante a été préparée :

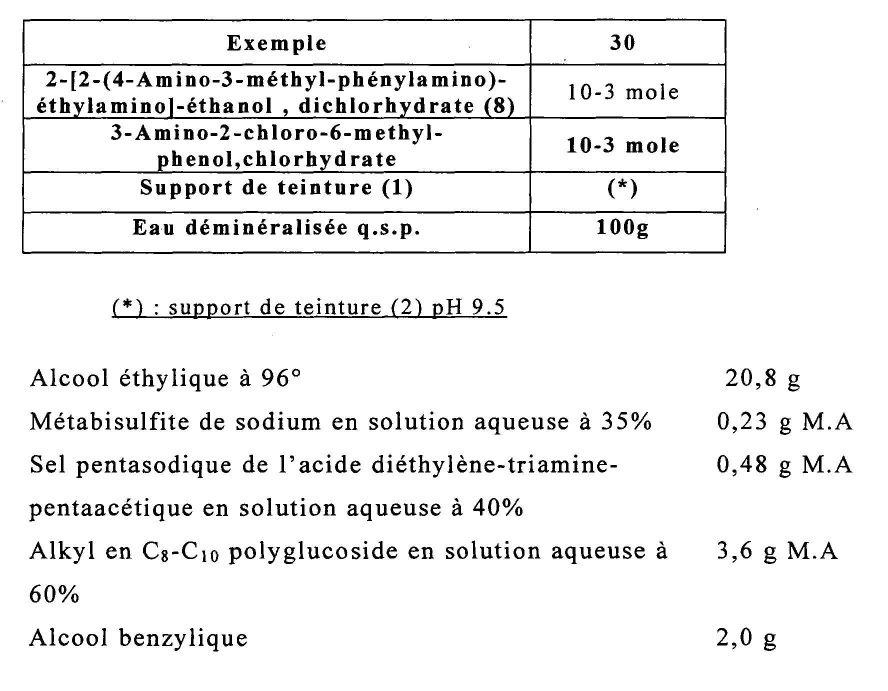

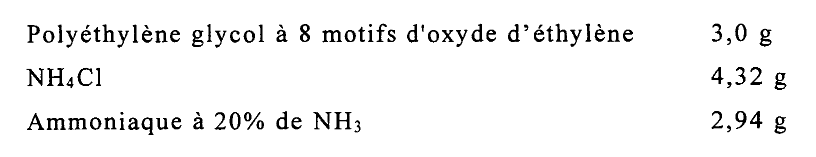

Au moment de l'emploi, chaque composition est mélangée avec un poids égal d'eau oxygénée à 20 volumes (6% en poids). On obtient un pH final de 9,5.

Chaque mélange obtenu est appliqué sur des mèches de cheveux gris à 90 % de blancs. Après 30 minutes de pause, les mèches sont rincées, lavées avec un shampooing standard, rincées à nouveau puis séchées.

La nuance obtenue figure dans le tableau ci-dessous :

| Exemple | 30 |
|---|---|
| Nuance observée | gris rouge |

### Exemples 31 à 42 : Composition tinctoriale à partir du 5-(4-Amino-2-methyl-phenylamino)-propan-1-ol, dichlorhydrate (24)

### - Exemples 31 à 37: Teinture en milieu acide

Les compositions tinctoriales suivantes sont préparées :

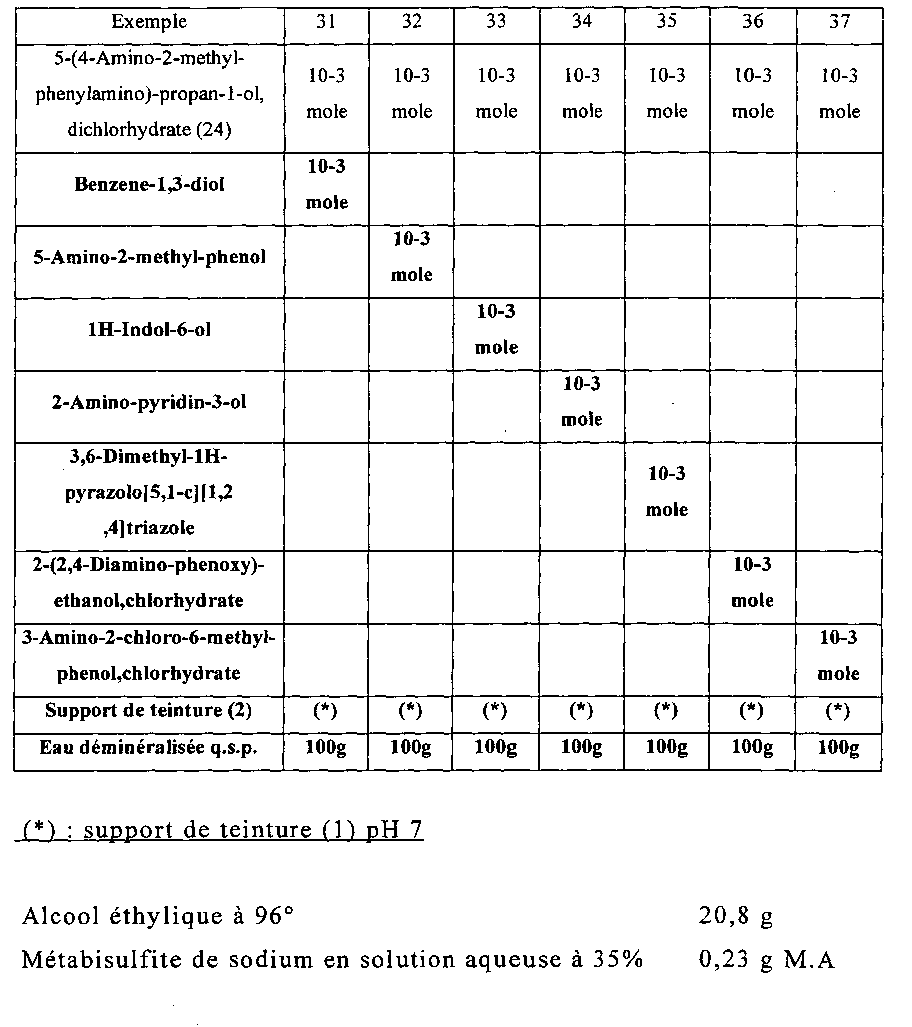

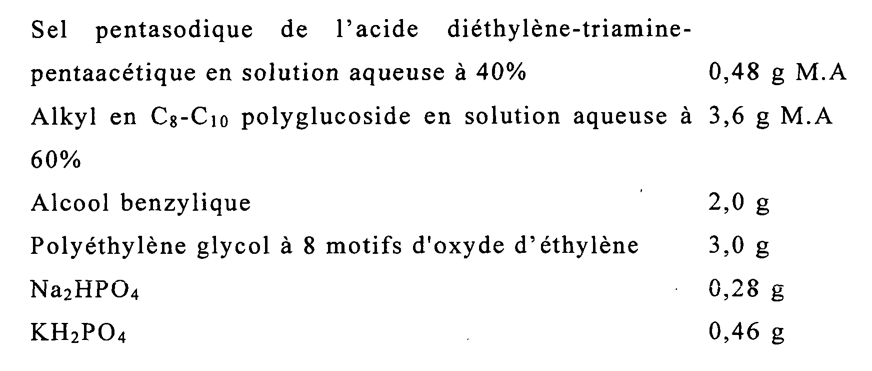

Au moment de l'emploi, chaque composition est mélangée avec un poids égal d'eau oxygénée à 20 volumes (6% en poids). On obtient un pH final de 7.

Chaque mélange obtenu est appliqué sur des mèches de cheveux gris à 90 % de blancs. Après 30 minutes de pause, les mèches sont rincées, lavées avec un shampooing standard, rincées à nouveau puis séchées.

Les nuances obtenues figurent dans le tableau ci-dessous :

| **Exemple** | **31** | **32** | **33** | **34** | **35** | **36** | **37** |
|---|---|---|---|---|---|---|---|
| **Nuance observée** | brun jaune | gris violet intense | gris intense | brun intense | rouge | bleu intense | violet-bleu intense |

### - Exemples 38 à : teinture en milieu basique

Les compositions tinctoriales suivantes sont préparées :

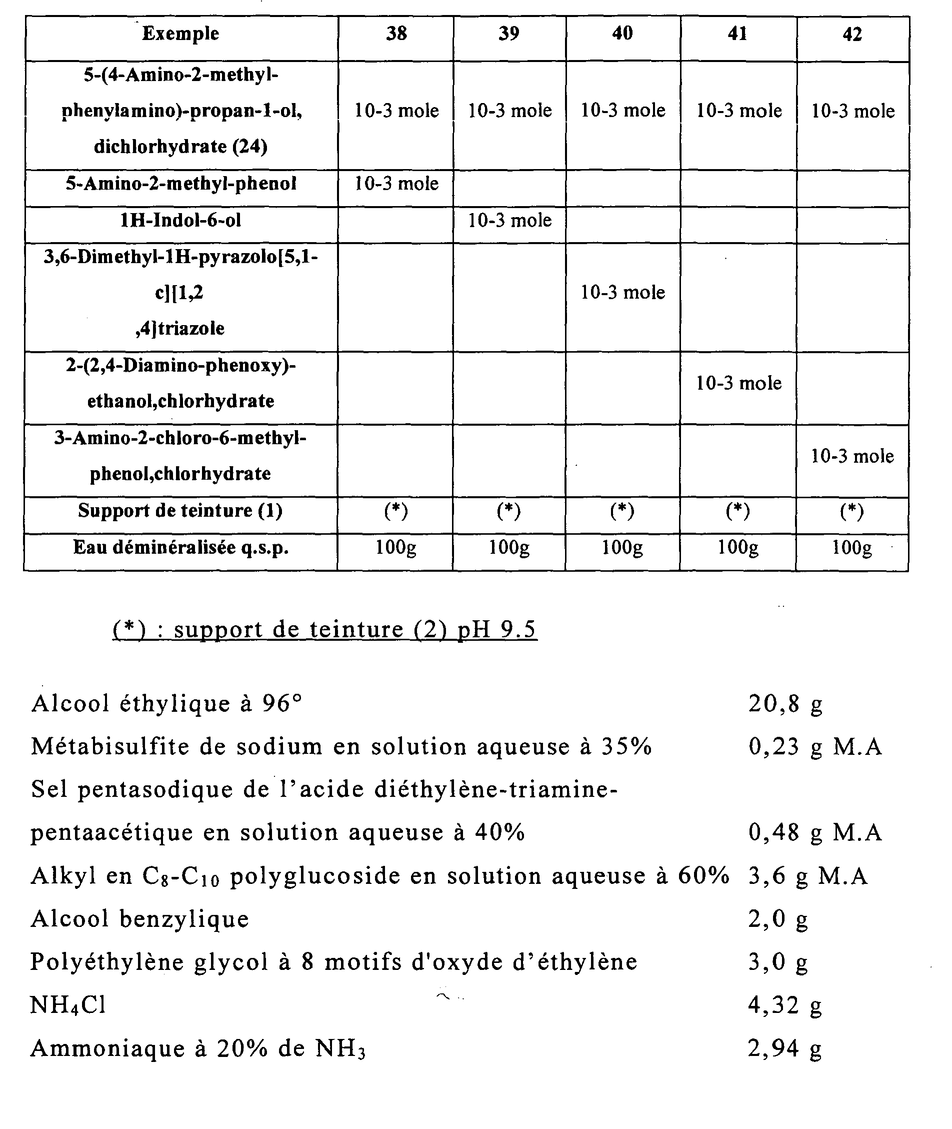

Au moment de l'emploi, chaque composition est mélangée avec un poids égal d'eau oxygénée à 20 volumes (6% en poids). On obtient un pH final de 9,5.

Chaque mélange obtenu est appliqué sur des mèches de cheveux gris à 90 % de blancs. Après 30 minutes de pause, les mèches sont rincées, lavées avec un shampooing standard, rincées à nouveau puis séchées.

Les nuances obtenues figurent dans le tableau ci-dessous :

| **Exemple** | **38** | **39** | **40** | **41** | **42** |
|---|---|---|---|---|---|
| **Nuance observée** | violet | orangé | rouge chromatique | bleu intense | violet-bleu intense |

### Exemples 43 à 55 : Composition tinctoriale à partir du 6-(4-Amino-2-méthyl-phénylamino)-hexan-1-ol, dichlorhydrate (12)

### - Exemples 43 à 49 : Teinture en milieu acide

Les compositions tinctoriales suivantes sont préparées :

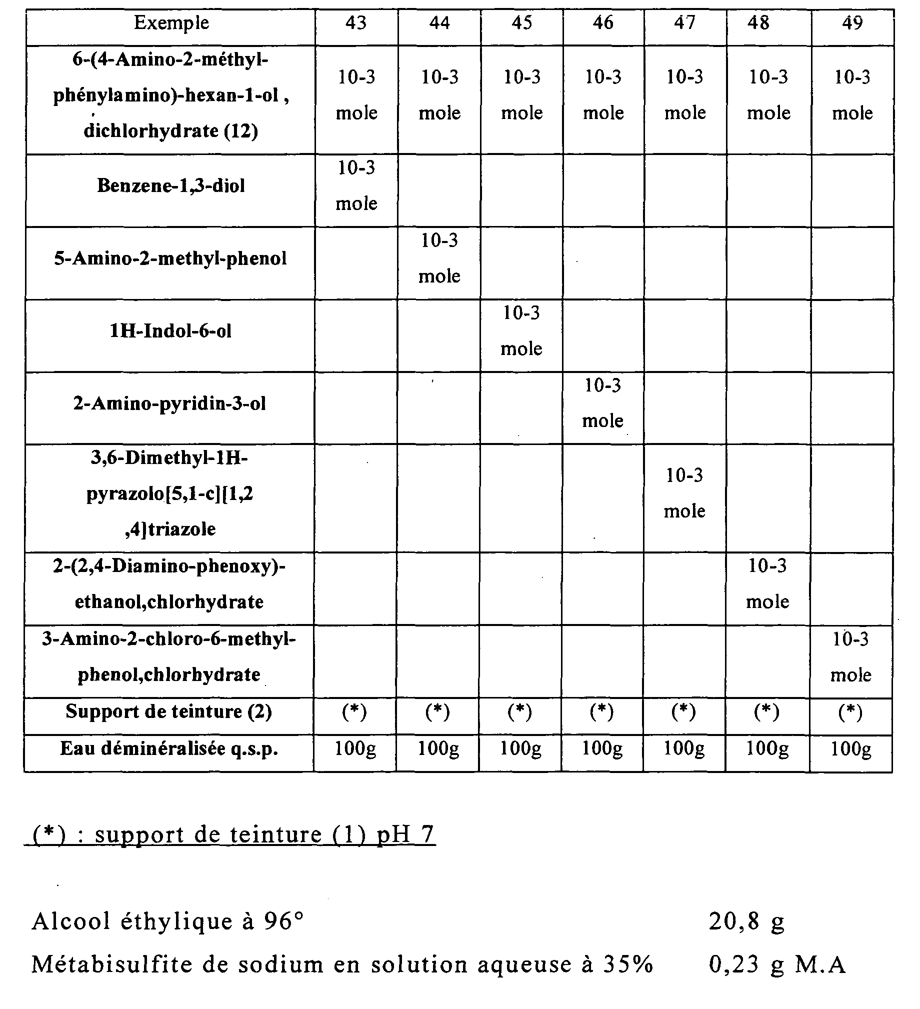

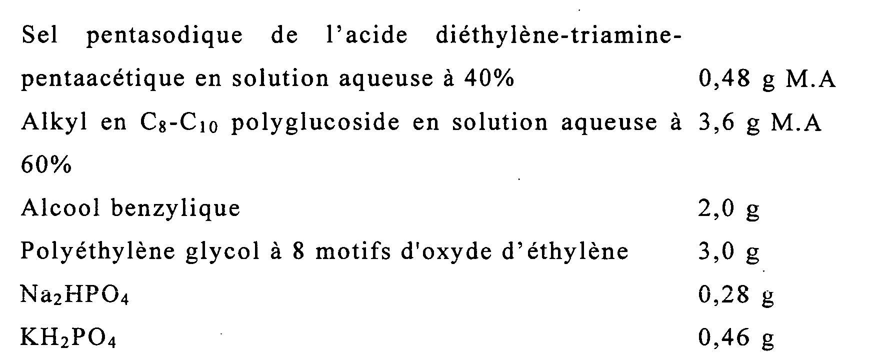

Au moment de l'emploi, chaque composition est mélangée avec un poids égal d'eau oxygénée à 20 volumes (6% en poids). On obtient un pH final de 7.

Chaque mélange obtenu est appliqué sur des mèches de cheveux gris à 90 % de blancs. Après 30 minutes de pause, les mèches sont rincées, lavées avec un shampooing standard, rincées à nouveau puis séchées.

Les nuances obtenues figurent dans le tableau ci-dessous :

| **Exemple** | **43** | **44** | **45** | **46** | **47** | **48** | **49** |
|---|---|---|---|---|---|---|---|
| **Nuance observée** | brun jaune | violet-bleu intense | gris intense | brun intense | rouge | bleu intense | violet-bleu intense |

### - Exemples 50 à 55 : teinture en milieu basique

Les compositions tinctoriales suivantes sont préparées :

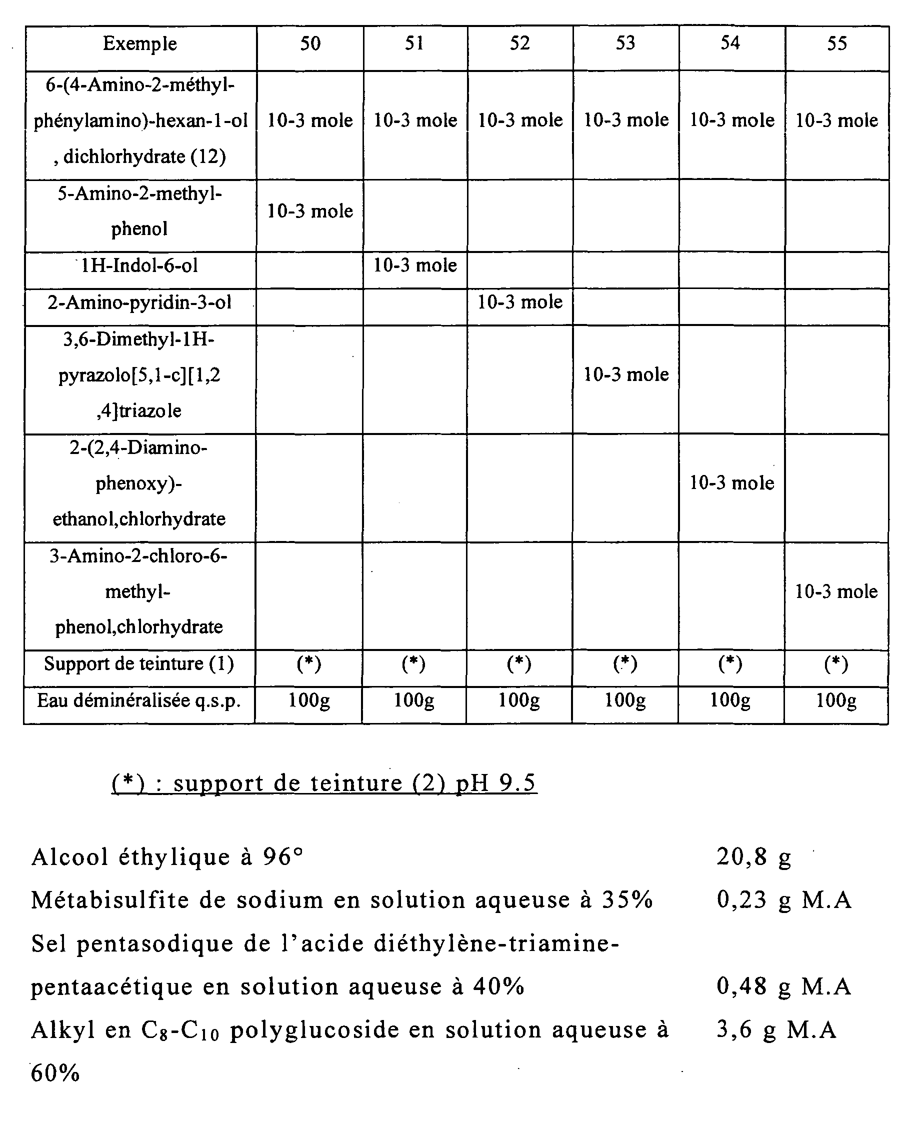

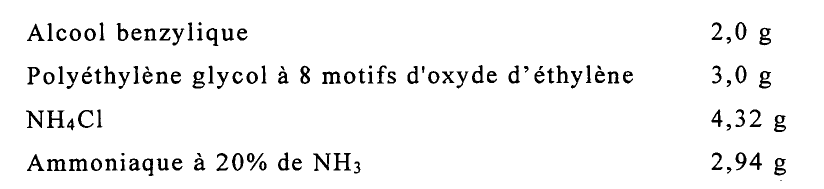

Au moment de l'emploi, chaque composition est mélangée avec un poids égal d'eau oxygénée à 20 volumes (6% en poids). On obtient un pH final de 9,5.

Chaque mélange obtenu est appliqué sur des mèches de cheveux gris à 90 % de blancs. Après 30 minutes de pause, les mèches sont rincées, lavées avec un shampooing standard, rincées à nouveau puis séchées.

Les nuances obtenues figurent dans le tableau ci-dessous :

| **Exemple** | **50** | **51** | **52** | **53** | **54** | **55** |
|---|---|---|---|---|---|---|
| **Nuance observée** | violet intense | orangé | brun rouge | violet-rouge chromati que | bleu intense | violet-bleu intense |

### Exemples 56 à 65 : Composition tinctoriale à partir du 5-(4-Amino-3-methyl-phenylamino)-pentan-1-ol, dichlorhydrate (18)

### - Exemples 55 à 61: Teinture en milieu acide

Les compositions tinctoriales suivantes sont préparées :

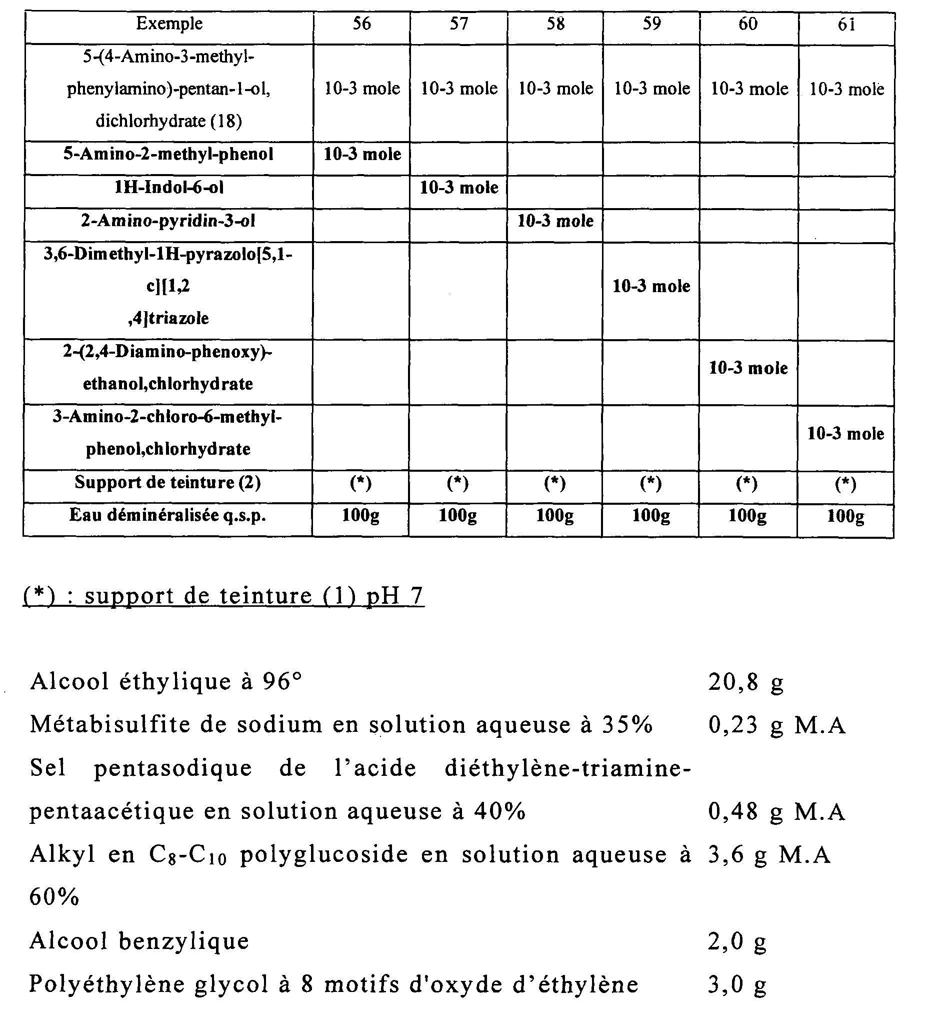

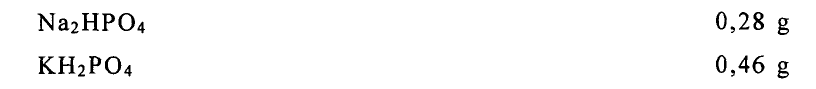

Au moment de l'emploi, chaque composition est mélangée avec un poids égal d'eau oxygénée à 20 volumes (6% en poids). On obtient un pH final de 7.

Chaque mélange obtenu est appliqué sur des mèches de cheveux gris à 90 % de blancs. Après 30 minutes de pause, les mèches sont rincées, lavées avec un shampooing standard, rincées à nouveau puis séchées.

Les nuances obtenues figurent dans le tableau ci-dessous :

| **Exemple** | **56** | **57** | **58** | **59** | **60** | **61** |
|---|---|---|---|---|---|---|
| **Nuance observée** | gris violet-bleu intense | brun rouge intense | brun | rouge | gris vert-bleu intense | bleu intense |

### - Exemples 62 à 65 : teinture en milieu basique

Les compositions tinctoriales suivantes sont préparées :

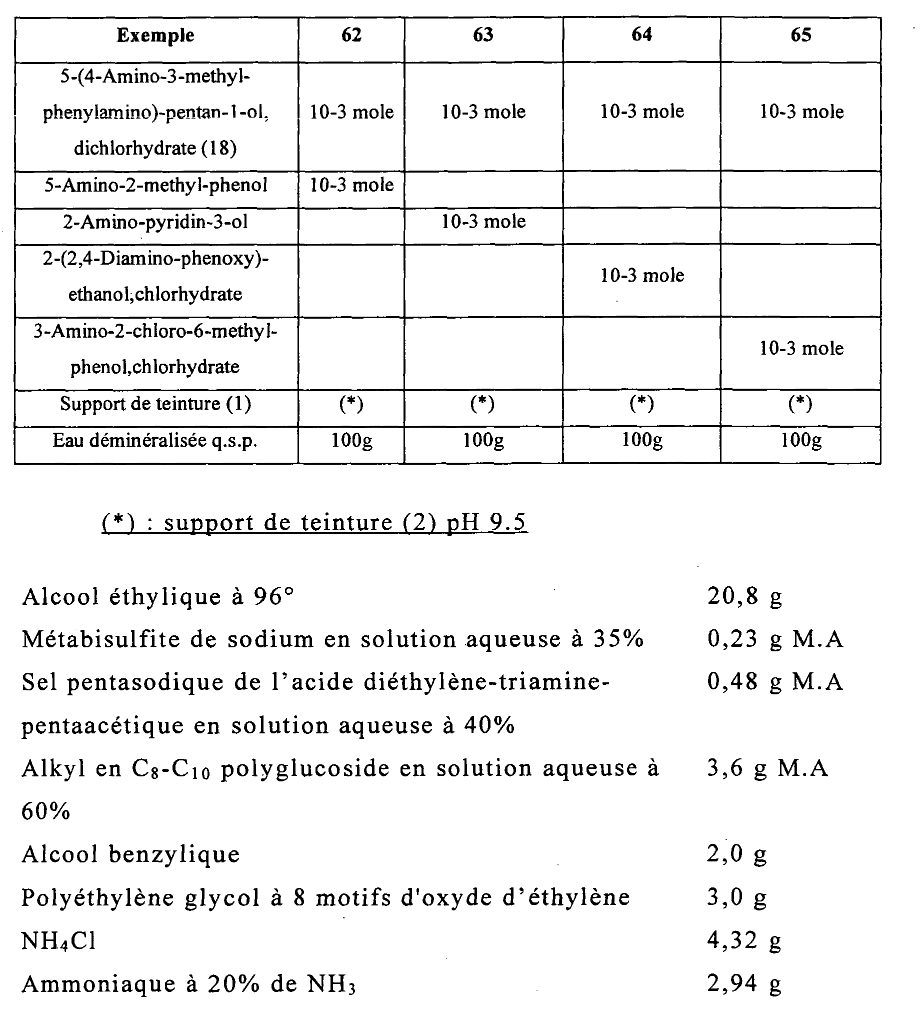

Au moment de l'emploi, chaque composition est mélangée avec un poids égal d'eau oxygénée à 20 volumes (6% en poids). On obtient un pH final de 9,5.

Chaque mélange obtenu est appliqué sur des mèches de cheveux gris à 90 % de blancs. Après 30 minutes de pause, les mèches sont rincées, lavées avec un shampooing standard, rincées à nouveau puis séchées.

Les nuances obtenues figurent dans le tableau ci-dessous :

| **Exemple** | **62** | **63** | **64** | **65** |
|---|---|---|---|---|
| **Nuance observée** | violet-bleu intense | orangé | vert-bleu intense | bleu chromatique intense |

### Exemples 66 à 78 : Composition tinctoriale à partir du 5-(4-Amino-2-methyl-phenylamino)-butan-1-ol, dichlorhydrate (22)

### - Exemples 66 à 72 : Teinture en milieu acide

Les compositions tinctoriales suivantes sont préparées :

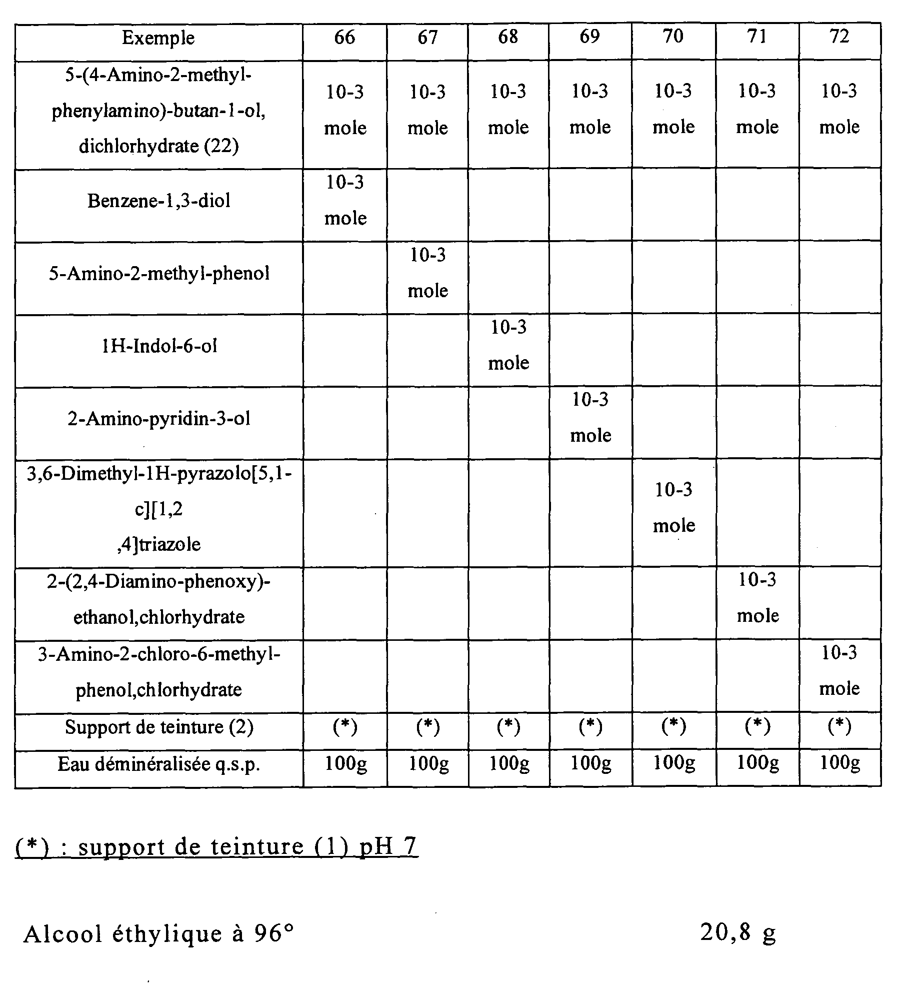

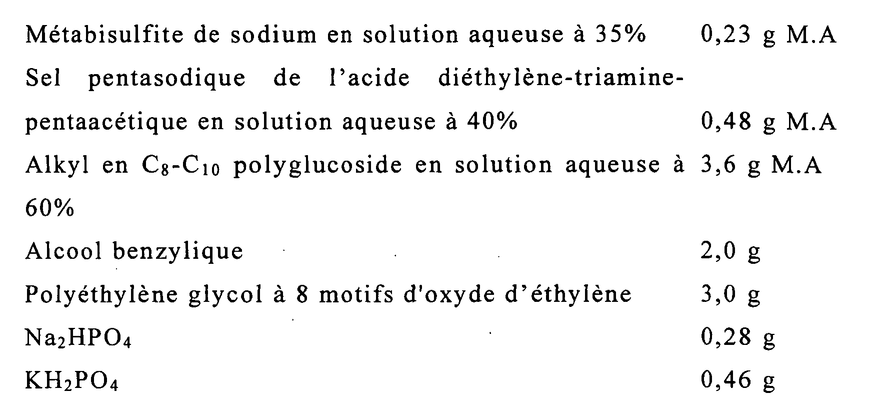

Au moment de l'emploi, chaque composition est mélangée avec un poids égal d'eau oxygénée à 20 volumes (6% en poids). On obtient un pH final de 7.

Chaque mélange obtenu est appliqué sur des mèches de cheveux gris à 90 % de blancs. Après 30 minutes de pause, les mèches sont rincées, lavées avec un shampooing standard, rincées à nouveau puis séchées.

Les nuances obtenues figurent dans le tableau ci-dessous :

| **Exemple** | **66** | **67** | **68** | **69** | **70** | **71** | **72** |
|---|---|---|---|---|---|---|---|
| **Nuance observée** | brun jaune | violet intense | gris intense | brun intense | rouge | bleu intense | violet-bleu intense |

### - Exemples 73 à 78 : teinture en milieu basique

Les compositions tinctoriales suivantes sont préparées :

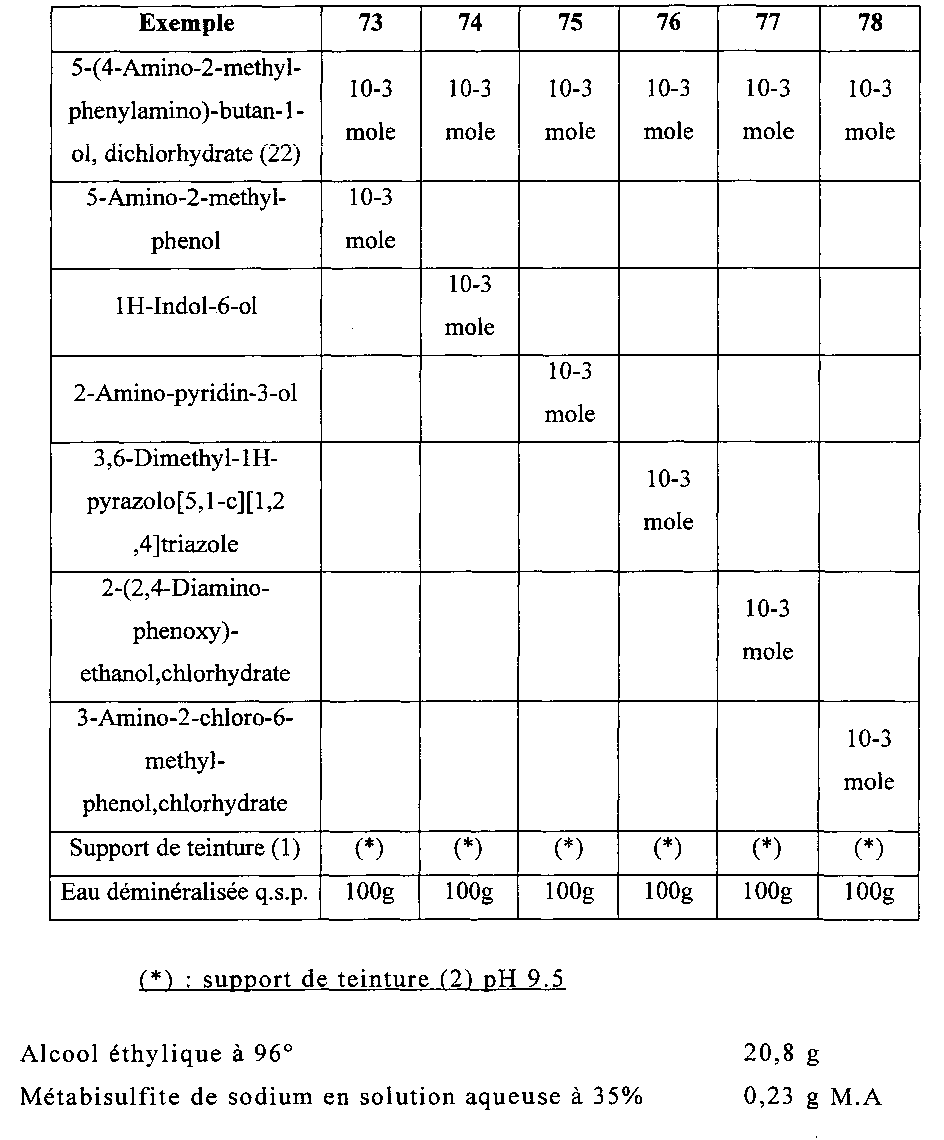

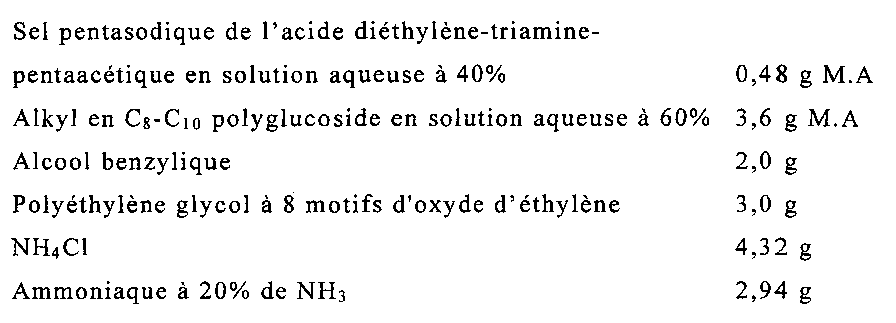

Au moment de l'emploi, chaque composition est mélangée avec un poids égal d'eau oxygénée à 20 volumes (6% en poids). On obtient un pH final de 9,5.

Chaque mélange obtenu est appliqué sur des mèches de cheveux gris à 90 % de blancs. Après 30 minutes de pause, les mèches sont rincées, lavées avec un shampooing standard, rincées à nouveau puis séchées.

Les nuances obtenues figurent dans le tableau ci-dessous :

| Exemple | 73 | 74 | 75 | 76 | 77 | 78 |
|---|---|---|---|---|---|---|
| Nuance observée | violet | orangé | brun orangé | rouge chromatique | bleu intense | violet-bleu intense |

### Exemples 79 à 86: Composition tinctoriale à partir du 6-(4-Amino-3-méthyl-phénylamino)-hexan-1-ol, dichlorhydrate (10)

### - Exemples 79 à 83 : Teinture en milieu acide

Les compositions tinctoriales suivantes sont préparées :

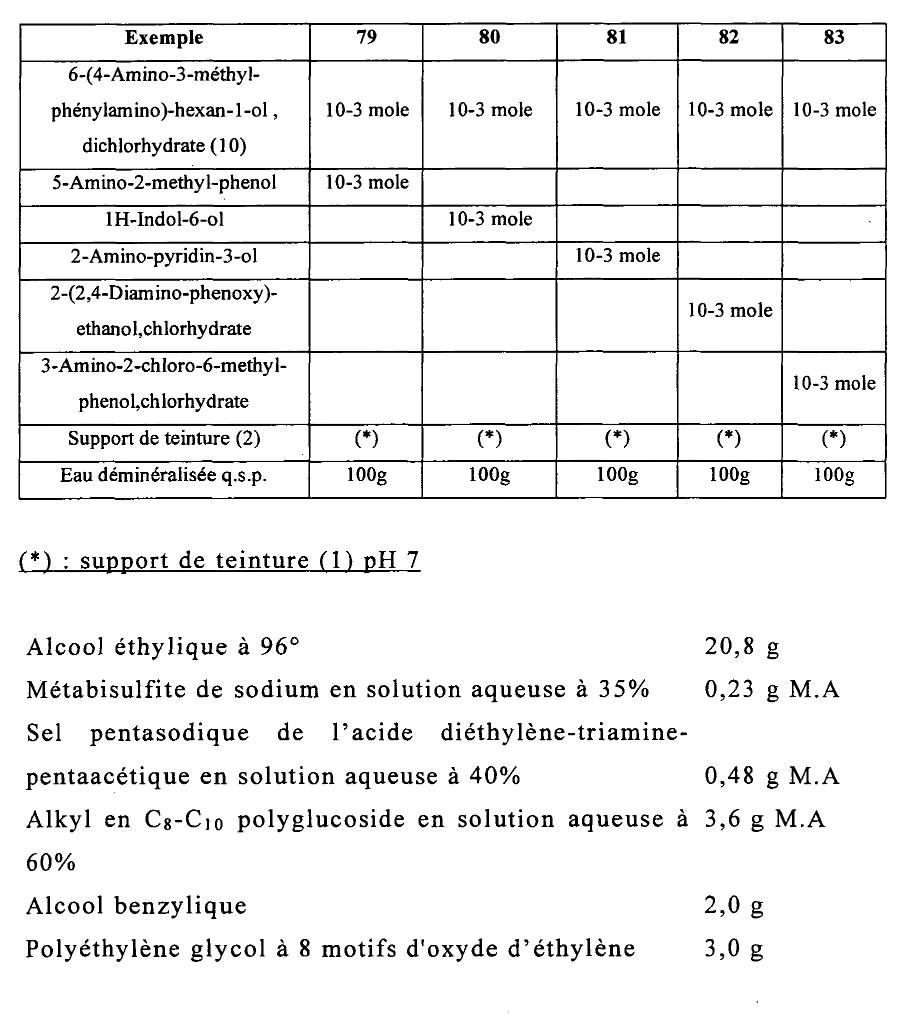

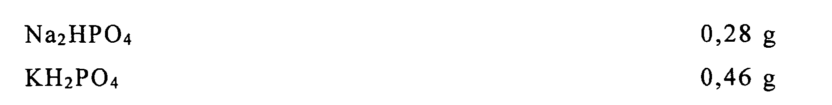

Au moment de l'emploi, chaque composition est mélangée avec un poids égal d'eau oxygénée à 20 volumes (6% en poids). On obtient un pH final de 7.

Chaque mélange obtenu est appliqué sur des mèches de cheveux gris à 90 % de blancs. Après 30 minutes de pause, les mèches sont rincées, lavées avec un shampooing standard, rincées à nouveau puis séchées.

Les nuances obtenues figurent dans le tableau ci-dessous :

| **Exemple** | **79** | **80** | **81** | **82** | **83** |
|---|---|---|---|---|---|
| **Nuance observée** | gris violet-bleu intense | brun intense | brun | gris vert-bleu intense | bleu intense |

### - Exemples 84 à 86 : teinture en milieu basique

Les compositions tinctoriales suivantes sont préparées :

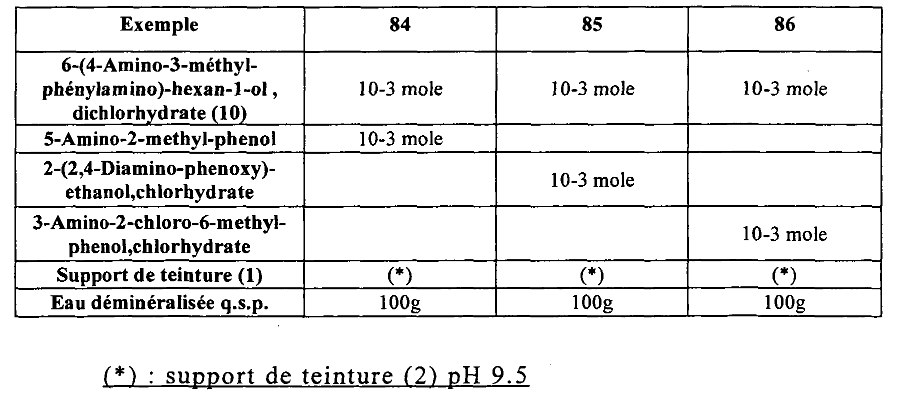

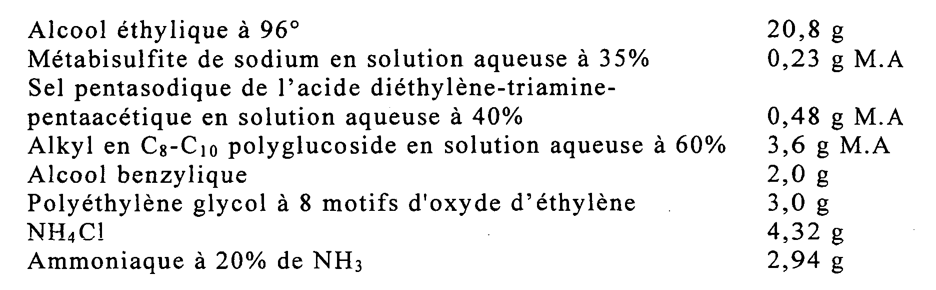

Au moment de l'emploi, chaque composition est mélangée avec un poids égal d'eau oxygénée à 20 volumes (6% en poids). On obtient un pH final de 9,5.

Chaque mélange obtenu est appliqué sur des mèches de cheveux gris à 90 % de blancs. Après 30 minutes de pause, les mèches sont rincées, lavées avec un shampooing standard, rincées à nouveau puis séchées.

Les nuances obtenues figurent dans le tableau ci-dessous :

| **Exemple** | **84** | **85** | **86** |
|---|---|---|---|
| **Nuance observée** | violet-bleu intense | vert-bleu | bleu |

### Exemples 87 à 99 : Composition tinctoriale à partir du 4-(4-Amino-2-methyl-phenylamino)-butan-2-ol, dichlorhydrate (28)

### - Exemples 87 à 93: Teinture en milieu acide

Les compositions tinctoriales suivantes sont préparées :

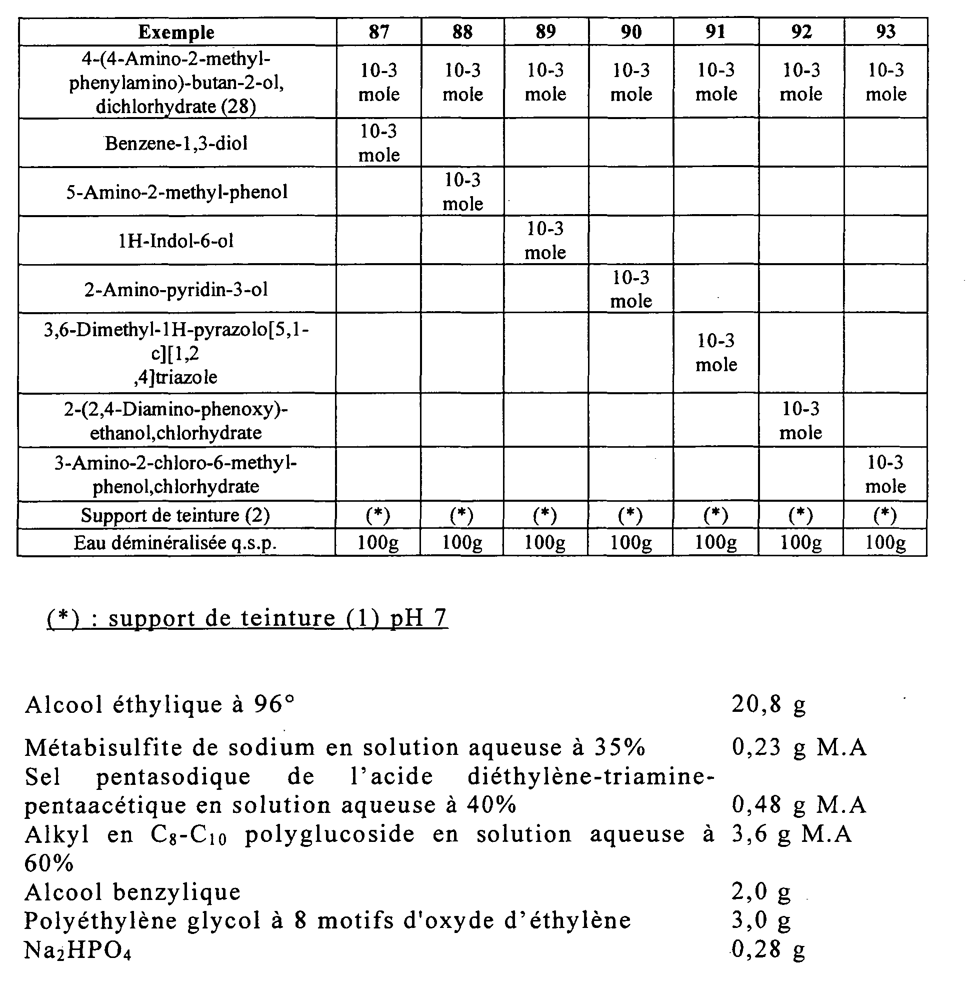

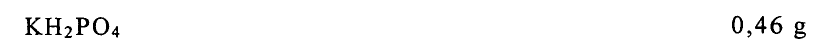

Au moment de l'emploi, chaque composition est mélangée avec un poids égal d'eau oxygénée à 20 volumes (6% en poids). On obtient un pH final de 7.

Chaque mélange obtenu est appliqué sur des mèches de cheveux gris à 90 % de blancs. Après 30 minutes de pause, les mèches sont rincées, lavées avec un shampooing standard, rincées à nouveau puis séchées.

Les nuances obtenues figurent dans le tableau ci-dessous :

| Exemple | 87 | 88 | 89 | 90 | 91 | 92 | 93 |
|---|---|---|---|---|---|---|---|
| Nuance observée | brun jaune | gris violet intense | intense | brun | orangé | bleu intense | violet-bleu intense |

### - Exemples 94 à 99: teinture en milieu basique

Les compositions tinctoriales suivantes sont préparées :

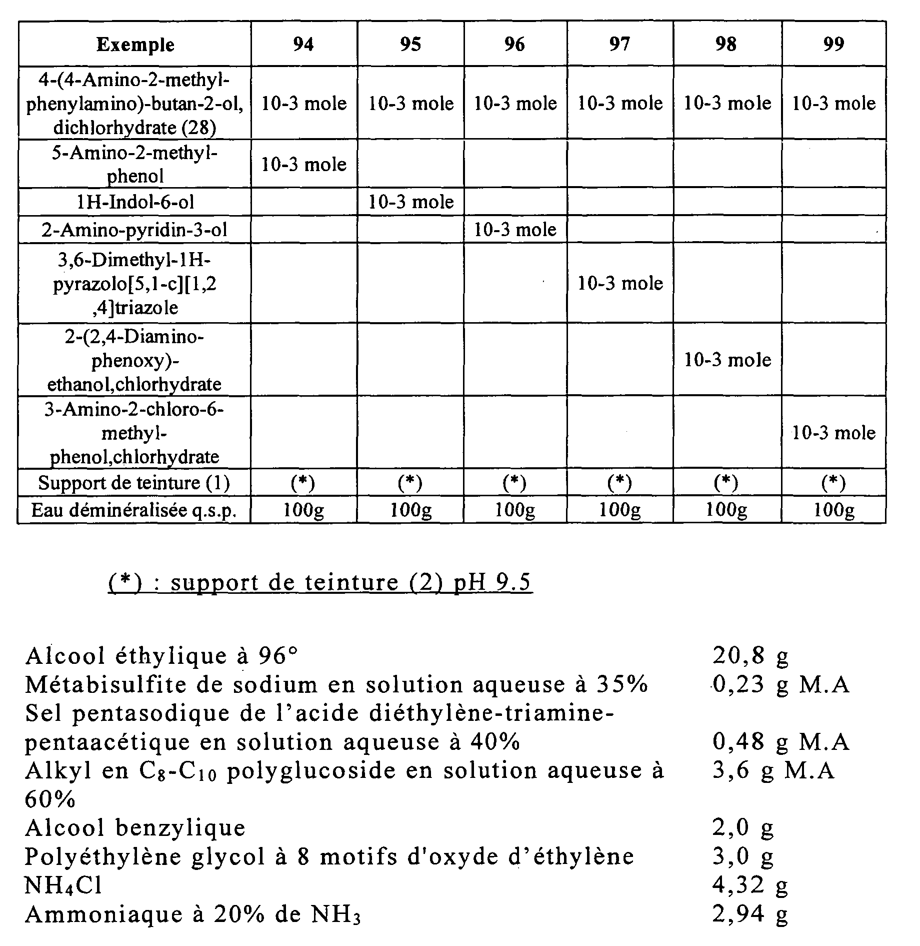

Au moment de l'emploi, chaque composition est mélangée avec un poids égal d'eau oxygénée à 20 volumes (6% en poids). On obtient un pH final de 9,5.

Chaque mélange obtenu est appliqué sur des mèches de cheveux gris à 90 % de blancs. Après 30 minutes de pause, les mèches sont rincées, lavées avec un shampooing standard, rincées à nouveau puis séchées.

Les nuances obtenues figurent dans le tableau ci-dessous :

| **Exemple** | **94** | **95** | **96** | **97** | **98** | **99** |
|---|---|---|---|---|---|---|
| **Nuance observée** | violet | orangé | orangé | rouge chromatique | bleu intense | violet-bleu intense |

### Exemples 100 à 111: Composition tinctoriale à partir du du 3-(4-Amino-2-methyl-phenylamino)-2,2-dimethyl-propan-1-ol, dichlorhydrate (26)

### - Exemples 100 à 106 : Teinture en milieu acide

Les compositions tinctoriales suivantes sont préparées :

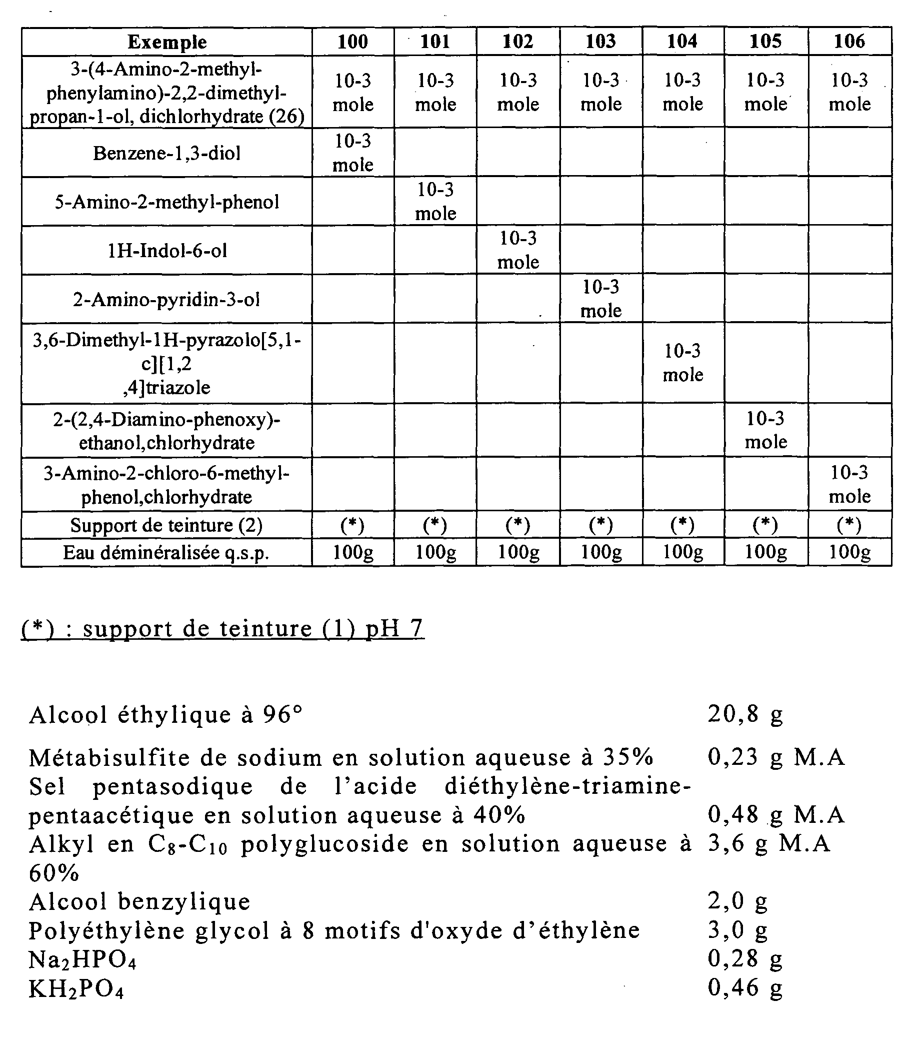

Au moment de l'emploi, chaque composition est mélangée avec un poids égal d'eau oxygénée à 20 volumes (6% en poids). On obtient un pH final de 7.

Chaque mélange obtenu est appliqué sur des mèches de cheveux gris à 90 % de blancs. Après 30 minutes de pause, les mèches sont rincées, lavées avec un shampooing standard, rincées à nouveau puis séchées.

Les nuances obtenues figurent dans le tableau ci-dessous :

| **Exemple** | **100** | **101** | **102** | **103** | **104** | **105** | **106** |
|---|---|---|---|---|---|---|---|
| **Nuance observée** | brun jaune | violet-bleu intense | brun rouge intense | gris intense | brun orangé | bleu intense | violet-bleu intense |

### - Exemples 107 à 111: teinture en milieu basique

Les compositions tinctoriales suivantes sont préparées :

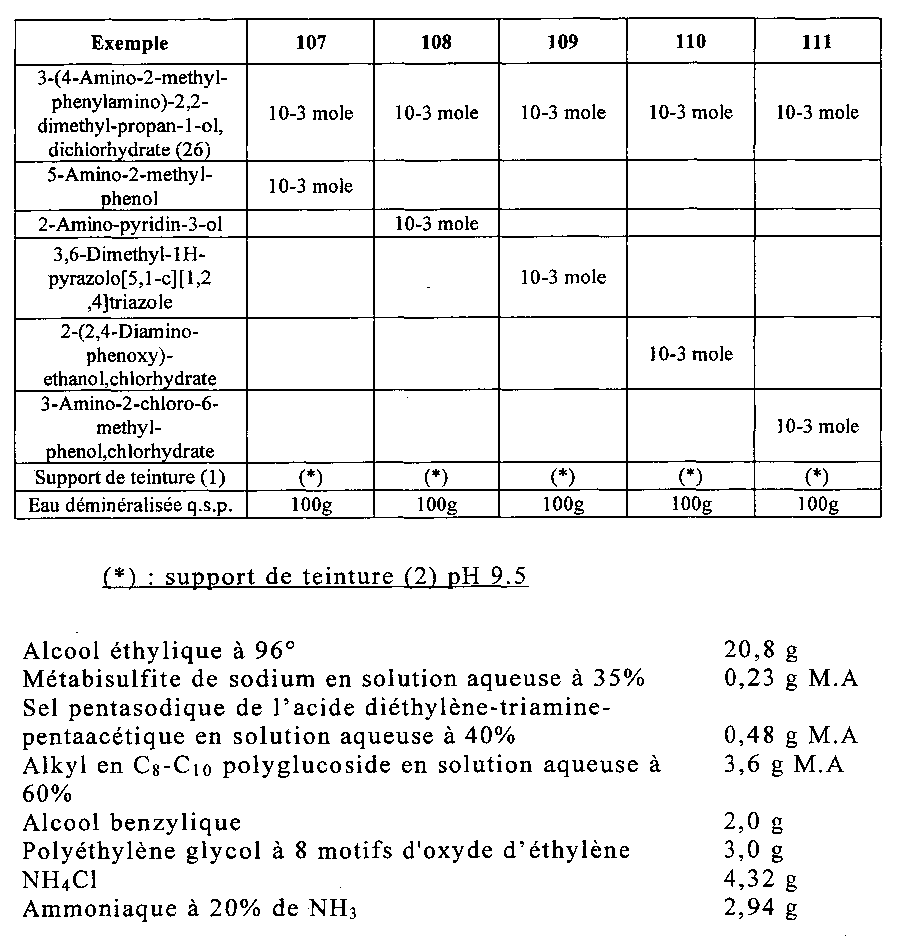

Au moment de l'emploi, chaque composition est mélangée avec un poids égal d'eau oxygénée à 20 volumes (6% en poids). On obtient un pH final de 9,5.

Chaque mélange obtenu est appliqué sur des mèches de cheveux gris à 90 % de blancs. Après 30 minutes de pause, les mèches sont rincées, lavées avec un shampooing standard, rincées à nouveau puis séchées.

Les nuances obtenues figurent dans le tableau ci-dessous :

| **Exemple** | **107** | **108** | **109** | **110** | **111** |
|---|---|---|---|---|---|
| **Nuance observée** | violet | brun rouge | violet-rouge chromatique | bleu intense | violet-bleu intense |

### Exemples 112 à 119 : Composition tinctoriale à partir du du 5-(4-Amino-3-methyl-phenylamino)-butan-1-ol, dichlorhydrate (30)

### - Exemples 112 à 116 : Teinture en milieu acide

Les compositions tinctoriales suivantes sont préparées :

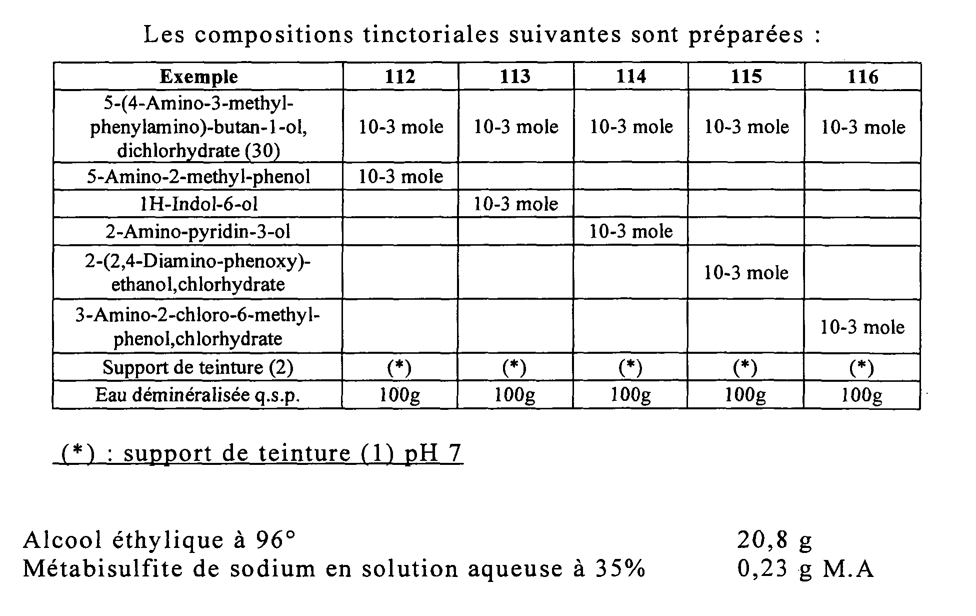

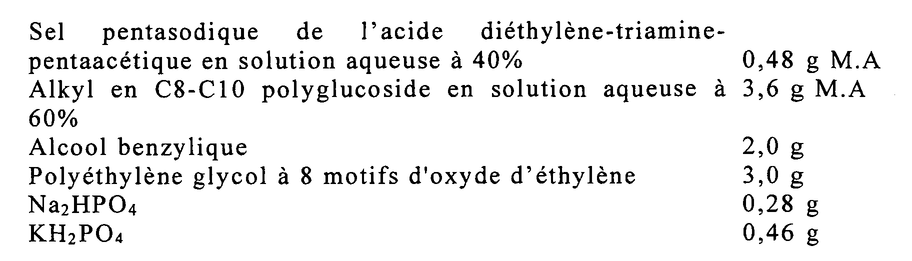

Au moment de l'emploi, chaque composition est mélangée avec un poids égal d'eau oxygénée à 20 volumes (6% en poids). On obtient un pH final de 7.

Chaque mélange obtenu est appliqué sur des mèches de cheveux gris à 90 % de blancs. Après 30 minutes de pause, les mèches sont rincées, lavées avec un shampooing standard, rincées à nouveau puis séchées.

Les nuances obtenues figurent dans le tableau ci-dessous :

| **Exemple** | **112** | **113** | **114** | **115** | **116** |
|---|---|---|---|---|---|
| **Nuance observée** | gris violet-bleu intense | brun rouge intense | brun | gris vert-bleu intense | bleu intense |

### - Exemples 117 à 119: teinture en milieu basique

Les compositions tinctoriales suivantes sont préparées :

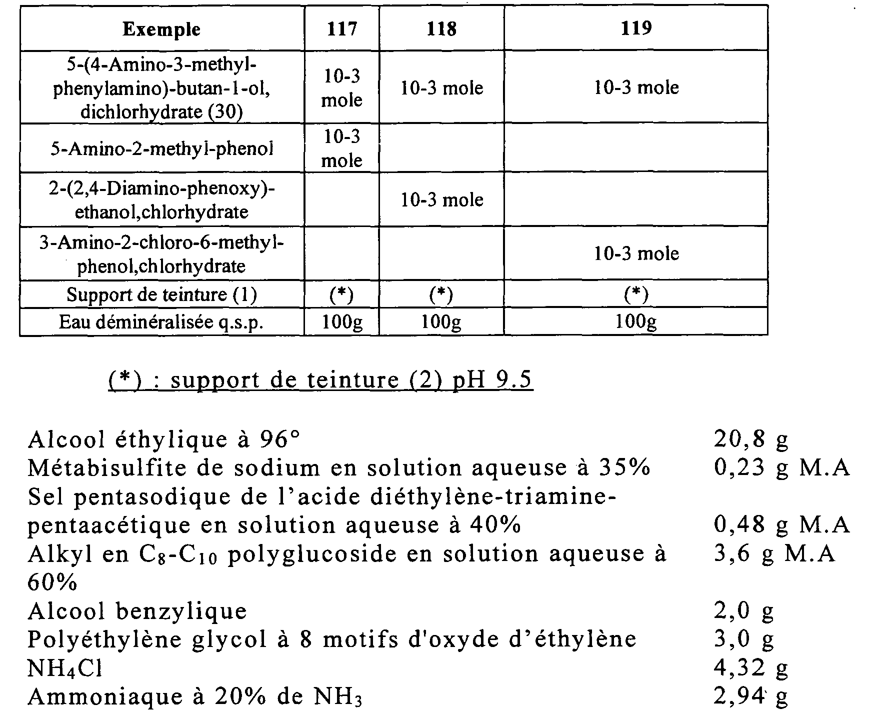

Au moment de l'emploi, chaque composition est mélangée avec un poids égal d'eau oxygénée à 20 volumes (6% en poids). On obtient un pH final de 9,5.

Chaque mélange obtenu est appliqué sur des mèches de cheveux gris à 90 % de blancs. Après 30 minutes de pause, les mèches sont rincées, lavées avec un shampooing standard, rincées à nouveau puis séchées.

Les nuances obtenues figurent dans le tableau ci-dessous :

| **Exemple** | **117** | **118** | **119** |
|---|---|---|---|
| **Nuance observée** | violet-bleu intense | vert-bleu intense | bleu chromatique intense |

### Exemples 120 à 129 : Composition tinctoriale à partir du 2-[2-(4-Amino-3-methyl-phenylamino)-ethoxy]-ethanol, dichlorhydrate (16)

### - Exemples 120 à 125 : Teinture en milieu acide

Les compositions tinctoriales suivantes sont préparées :

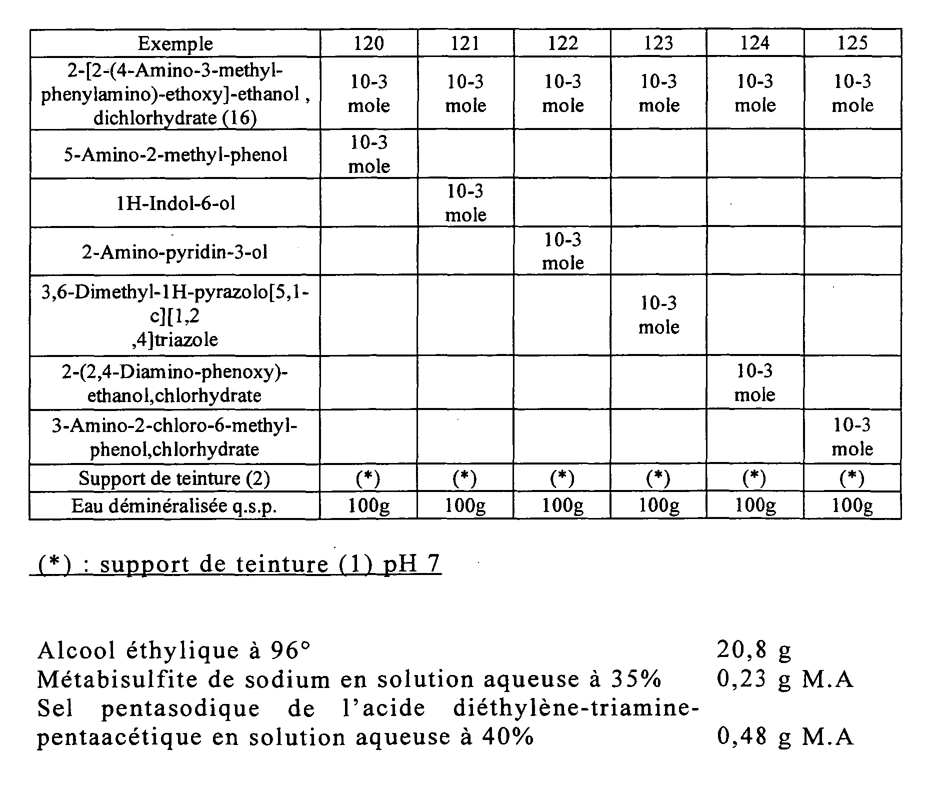

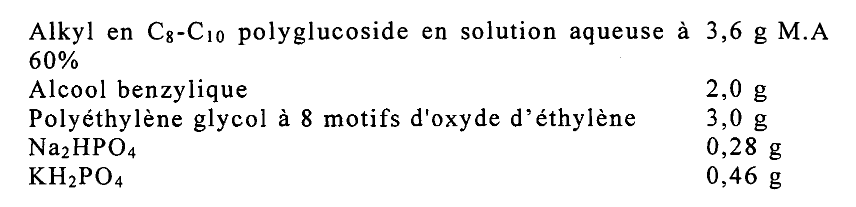

Au moment de l'emploi, chaque composition est mélangée avec un poids égal d'eau oxygénée à 20 volumes (6% en poids). On obtient un pH final de 7.

Chaque mélange obtenu est appliqué sur des mèches de cheveux gris à 90 % de blancs. Après 30 minutes de pause, les mèches sont rincées, lavées avec un shampooing standard, rincées à nouveau puis séchées.

Les nuances obtenues figurent dans le tableau ci-dessous :

| **Exemple** | **120** | **121** | **122** | **123** | **124** | **125** |
|---|---|---|---|---|---|---|
| **Nuance observée** | gris violet intense | orangé | brun orangé | orangé | gris vert-bleu intense | violet-bleu intense |

### - Exemples 126 à 129 : teinture en milieu basique

Les compositions tinctoriales suivantes sont préparées :

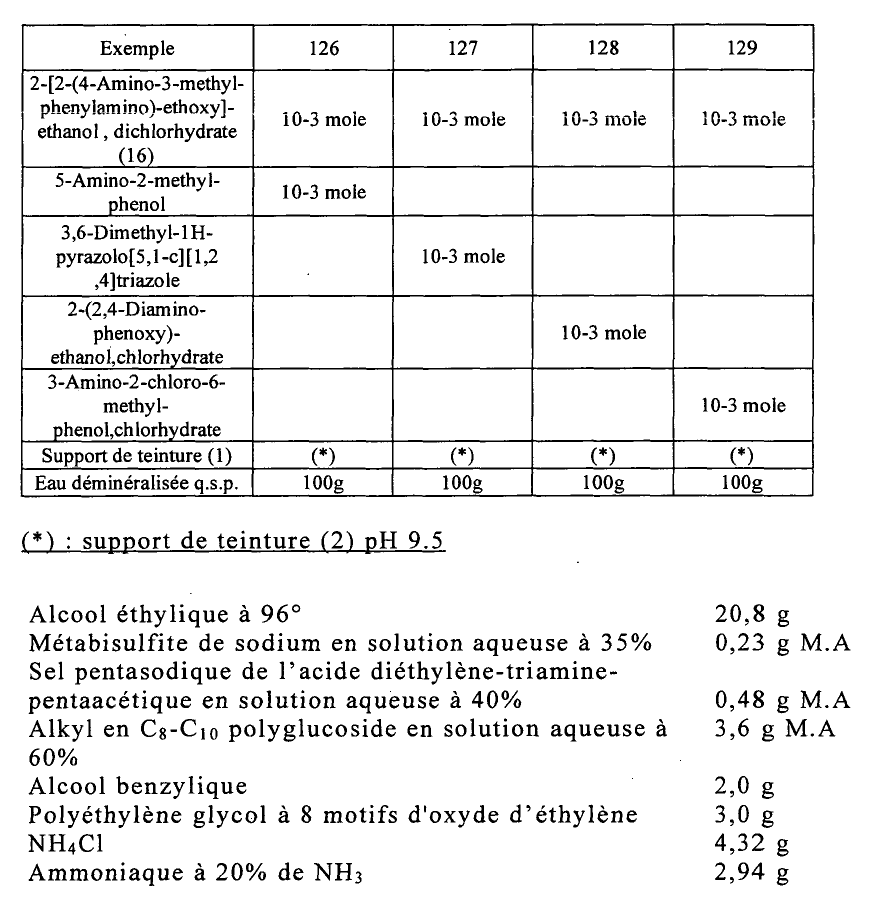

Au moment de l'emploi, chaque composition est mélangée avec un poids égal d'eau oxygénée à 20 volumes (6% en poids). On obtient un pH final de 9,5.

Chaque mélange obtenu est appliqué sur des mèches de cheveux gris à 90 % de blancs. Après 30 minutes de pause, les mèches sont rincées, lavées avec un shampooing standard, rincées à nouveau puis séchées.

Les nuances obtenues figurent dans le tableau ci-dessous :

| Exemple | 126 | 127 | 128 | 129 |
|---|---|---|---|---|
| Nuance observée | gris violet | rouge chromatique | vert-bleu | bleu intense |

### Exemples 130 à 138 : Composition tinctoriale à partir du 4-(4-Amino-3-methyl-phenylamino)-butan-2-ol, dichlorhydrate (32)

### - Exemples 130 à 134: Teinture en milieu acide

Les compositions tinctoriales suivantes sont préparées :

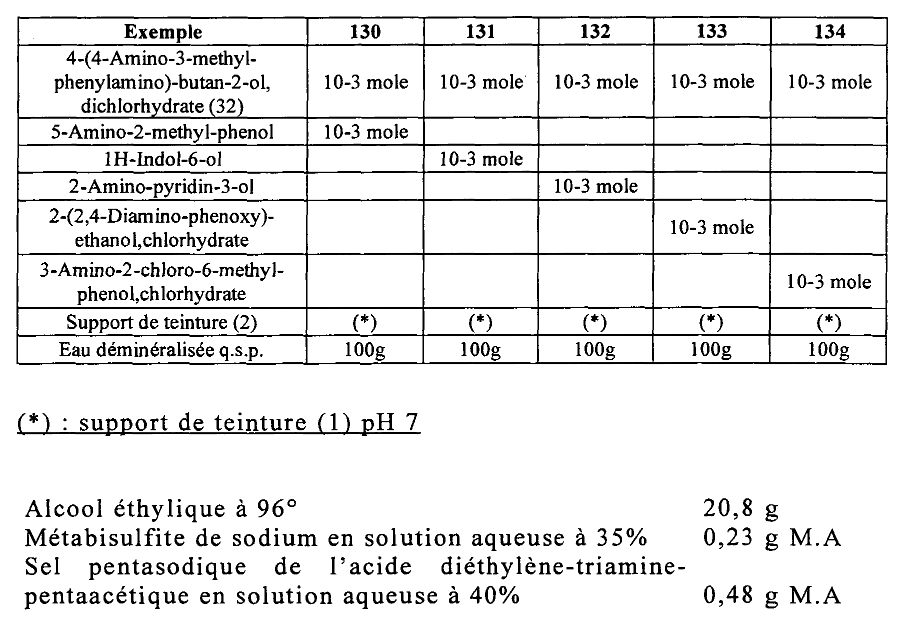

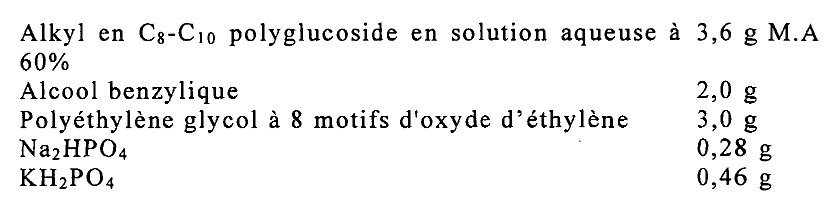

Au moment de l'emploi, chaque composition est mélangée avec un poids égal d'eau oxygénée à 20 volumes (6% en poids). On obtient un pH final de 7.

Chaque mélange obtenu est appliqué sur des mèches de cheveux gris à 90 % de blancs. Après 30 minutes de pause, les mèches sont rincées, lavées avec un shampooing standard, rincées à nouveau puis séchées.

Les nuances obtenues figurent dans le tableau ci-dessous :

| **Exemple** | **130** | **131** | **132** | **133** | **134** |
|---|---|---|---|---|---|
| **Nuance observée** | gris violet intense | brun orangé | brun jaune | gris vert-bleu intense | bleu intense |

### - Exemples 135 à 138 : teinture en milieu basique

Les compositions tinctoriales suivantes sont préparées :

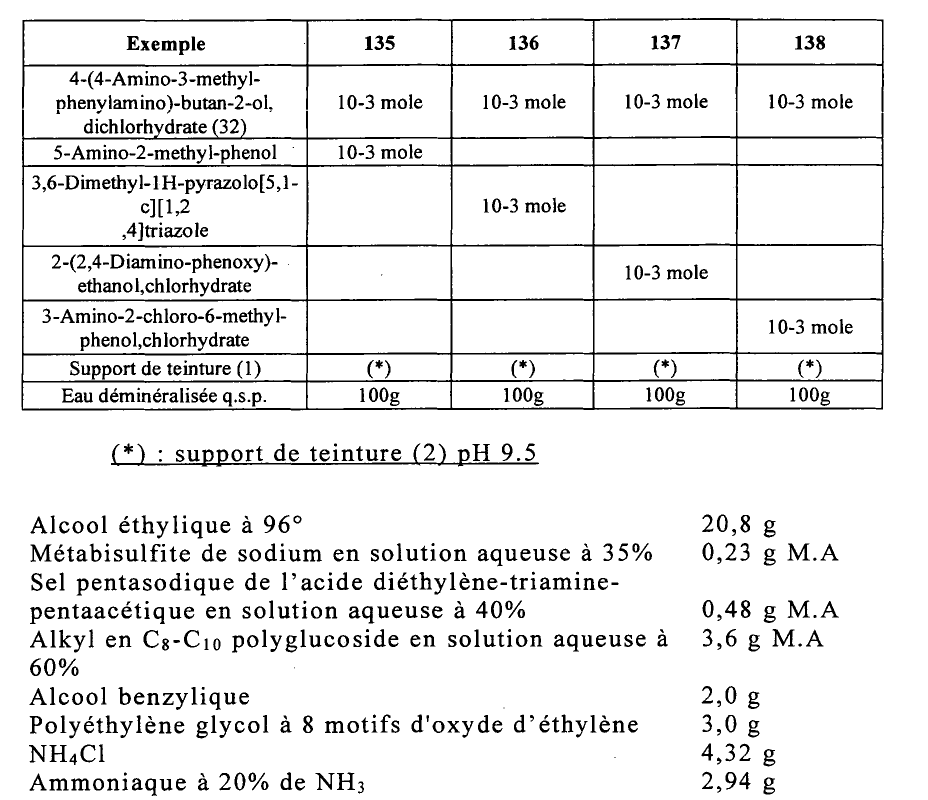

Au moment de l'emploi, chaque composition est mélangée avec un poids égal d'eau oxygénée à 20 volumes (6% en poids). On obtient un pH final de 9,5.

Chaque mélange obtenu est appliqué sur des mèches de cheveux gris à 90 % de blancs. Après 30 minutes de pause, les mèches sont rincées, lavées avec un shampooing standard, rincées à nouveau puis séchées.

Les nuances obtenues figurent dans le tableau ci-dessous :

| **Exemple** | **135** | **136** | **137** | **138** |
|---|---|---|---|---|
| Nuance observée | gris violet-bleu intense | rouge chromatique | vert-bleu intense | bleu intense |

## Revendications

1. Composé **caractérisé par le fait qu'**il s'agit d'une para-phénylènediamine secondaire N-alkylhydroxylée ortho-substituée et/ou méta-substituée de formule générale (I) : 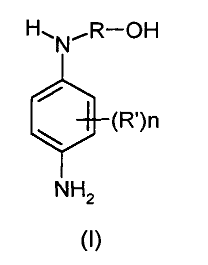 dans laquelle :
le radical R représente un radical alkylène en C₃-C₁₀, linéaire ou ramifié, éventuellement substitué par un ou plusieurs groupements choisis parmi les groupements amino, monoalkylamino, dialkylamino, alkylcarbonyle, amido, monoalkylaminocarbonyle, dialkylaminocarbonyle, éventuellement interrompu par un ou plusieurs atomes choisis parmi l'azote et l'oxygène,
le radical R' représente un radical choisi parmi les radicaux alkyle, alcoxy, hydroxyalcoxy, alcoxyalkyle, monohydroxyalkyle, polyhydroxyalkyle et l'atome de chlore,
n est un entier de 1 à 4.

2. Composé de formule (I) selon la revendication 1 dans laquelle R représente un radical alkylène linéaire ou ramifié en C₃-C₈, de préférence en C₃-C₆.

3. Composé de formule (I) selon la revendication 1 dans laquelle R représente un radical alkylène linéaire ou ramifié en C₃-C₆, de préférence en C₃-C₄ interrompu par un atome choisi parmi l'azote et l'oxygène.

4. Composé de formule (I) selon l'une des revendications 1 à 3 dans laquelle R' est un groupement choisi parmi les groupes alkyles en C₁-C₃ et alcoxy en C₁-C₃.

5. Composé de formule (I) selon la revendication 4 dans laquelle R' représente un groupe méthyle.

6. Composé de formule (I) selon l'une des revendications 1 à 5 dans laquelle n est égal à 1 ou 2.

7. Composé de formule (I) selon la revendication 6 dans laquelle n est égal à 1.

8. Composé de formule (I) selon l'une des revendications précédentes, **caractérisé par le fait qu'**il est choisi parmi :
2-[2-(4-Amino-2-méthyl-phénylamino)-ethoxy]-ethanol ;
2-[2-(4-Amino-2-méthyl-phénylamino)-ethylamino]-ethanol ;
3-(4-Amino-2-méthyl-phénylamino)-propan-1-ol ;
2-(4-Amino-2-méthyl-phénylamino)-hexan-1-ol ;
1-(4-Amino-2-méthyl-phénylamino)-butan-2-ol ;
3-(4-Amino-2-méthyl-phénylamino)-2,2-diméthyl-propan-1-ol ;
6-(4-Amino-2-méthyl-phénylamino)-hexan-1-ol ;
4-(4-Amino-2-méthyl-phénylamino)-butan-1-ol ;
2-(4-Amino-2-méthyl-phénylamino)-3, 3-diméthyl-butan-1-ol ;
2-(4-Amino-2-méthyl-phénylamino)-3-méthyl-butan-1-ol ;
2-(4-Amino-2-méthyl-phénylamino)-4-méthyl-pentan-1-ol ;
2-(4-Amino-2-méthyl-phénylamino)-propan-1-ol ;
5-(4-Amino-2-méthyl-phénylamino)-pentan-1-ol ;
2-(4-Amino-2-méthyl-phénylamino)-pentan-1-ol ;
2-(4-Amino-2-méthyl-phénylamino)-butan-1-ol ;
4-(4-Amino-2-méthyl-phénylamino)-butan-2-ol ;
2-(4-Amino-2-méthyl-phénylamino)-3-méthyl-pentan-1-ol ;
1-(4-Amino-2-méthyl-phénylamino)-propan-2-ol ;
2-(4-Amino-2-méthyl-phénylamino)-2-méthyl-propan-1-ol ;
2-[2-(4-Amino-3-méthyl-phénylamino)-éthoxy]-éthanol ;
2-[2-(4-Amino-3-méthyl-phénylamino)-éthylamino]-éthanol ;
3-(4-Amino-3-méthyl-phénylamino)-propan-1-ol ;
2-(4-Amino-3-méthyl-phénylamino)-hexan-1-ol ;
1-(4-Amino-3-méthyl-phénylamino)-butan-2-ol ;
3-(4-Amino-3-méthyl-phénylamino)-2,2-diméthyl-propan-1-ol ;
6-(4-Amino-3-méthyl-phénylamino)-hexan-1-ol ;
4-(4-Amino-3-méthyl-phénylamino)-butan-1-ol
2-(4-Amino-3-méthyl-phénylamino)-3,3-diméthyl-butan-1-ol ;
2-(4-Amino-3-méthyl-phénylamino)-3-méthyl-butan-1-ol ;
2-(4-Amino-3-méthyl-phénylamino)-4-méthyl-pentan-1-ol ;
2-(4-Amino-3-méthyl-phénylamino)-propan-1-ol ;
5-(4-Amino-3-méthyl-phénylamino)-pentan-1-ol;
2-(4-Amino-3-méthyl-phénylamino)-pentan-1-ol ;
2-(4-Amino-3-méthyl-phénylamino)-butan-1-ol;
4-(4-Amino-3-méthyl-phénylamino)-butan-2-ol ;
2-(4-Amino-3-méthyl-phénylamino)-3-méthyl-pentan-1-ol ;
1-(4-Amino-3-méthyl-phénylamino)-propan-2-ol ;
2-(4-Amino-3-méthyl-phénylamino)-2-méthyl-propan-1-ol
N-1-(2-amino-éthyl)-2-méthoxyméthyl-benzène-1,4-diamine.

9. Composé de formule (I) selon l'une des revendications précédentes, **caractérisé par le fait que** ce composé se présente sous la forme d'un sel.

10. Composé de formule (I) selon la revendication 9, **caractérisé par le fait qu'**il se présente sous la forme d'un sel d'addition avec un acide choisi parmi les chlorhydrates, les bromhydrates, les sulfates, les citrates, les succinates, les tartrates, les lactates, les tosylates, les benzènesulfonates, les phosphates et les acétates .

11. Procédé de préparation du composé de formule (I) selon l'une des revendications 1 à 10, **caractérisé par le fait que** l'on effectue une étape de réduction d'un composé nitré correspondant.

12. Utilisation d'un composé de formule générale (I) selon l'une des revendications 1 à 10 pour la teinture des fibres, de préférence les fibres kératiniques telles que les cheveux.

13. Composition cosmétique pour la teinture des fibres, de préférence les fibres kératiniques telles que les cheveux, comprenant, dans un milieu approprié pour la teinture, au moins un composé de formule générale (I) selon l'une des revendications 1 à 10.

14. Composition selon la revendication 13 **caractérisée par le fait que** la concentration du composé de formule générale (I) est comprise entre 0,0001 et 20%, de préférence entre 0,005 à 6% en poids par rapport au poids total de la composition

15. Composition selon l'une des revendications 13 ou 14 **caractérisée par le fait que** le milieu approprié pour la teinture est constitué par de l'eau ou par un mélange d'eau et d'au moins un solvant organique tel que, les alcools inférieurs en C₁-C₄, ramifiés ou non, tels que l'éthanol et l'isopropanol ; les polyols et éthers de polyols comme le 2-butoxyéthanol, le propylèneglycol, le monométhyléther de propylèneglycol, le monoéthyléther et le monométhyléther du diéthylèneglycol, le glycérol ainsi que les alcools aromatiques comme l'alcool benzylique ou le phénoxyéthanol, et leurs mélanges.

16. Composition selon l'une des revendications 13 à 15, **caractérisée par le fait qu'**elle comprend au moins un adjuvant cosmétique choisi dans le groupe formé par les agents anti-oxydants, les agents de pénétration, les agents séquestrants, les parfums, les tampons, les agents dispersants, les tensioactifs, les agents conditionneurs, les agents filmogènes, les polymères, les céramides, les agents conservateurs, les agents nacrants ou opacifiants, les vitamines ou provitamines.

17. Composition selon la revendication 16, **caractérisée par le fait que** la quantité en adjuvant cosmétique est comprise pour chacun d'eux entre 0,01 et 20 % en poids par rapport au poids de la composition.

18. Composition selon l'une des revendications 13 à 17, **caractérisée par le fait qu'**elle comprend au moins un précurseur de colorant d'oxydation additionnel différent des composés de formule (I) selon l'une des revendications 1 à 10.

19. Composition selon la revendication 18, **caractérisée par le fait que** le précurseur de colorant d'oxydation additionnel est un coupleur d'oxydation choisi parmi les méta-phénylènediamines, les méta-aminophénols, les méta-diphénols, les coupleurs naphtaléniques, les coupleurs hétérocycliques ainsi que leurs sels d'addition.

20. Composition selon la revendication 19, **caractérisée par le fait que** la concentration de ce coupleur est comprise entre 0,0001 et 20 % en poids par rapport au poids total de la composition.

21. Composition selon l'une des revendications 18 à 20, **caractérisée par le fait que** le précurseur de colorant d'oxydation additionnel est une base d'oxydation différente des composés de formule (I) selon l'une des revendications 1 à 10, choisie parmi les para-phénylènediamines, les bis-phénylalkylènediamines, les para-aminophénols, les ortho-aminophénols, les bases hétérocycliques et leurs sels d'addition.

22. Composition selon la revendication 21, **caractérisée par le fait que** la concentration de cette base d'oxydation est comprise entre 0,0001 et 20% en poids par rapport au poids total de la composition.

23. Composition selon l'une des revendications 13 à 22, **caractérisée par le fait qu'**elle renferme un ou plusieurs colorants directs naturels ou cationiques.

24. Composition prête à l'emploi **caractérisée en ce qu'**il s'agit d'un mélange d'une composition selon l'une des revendications 13 à 23 avec au moins un agent oxydant choisi parmi le peroxyde d'hydrogène, le peroxyde d'urée, les bromates de métaux alcalins, les persels, les peracides et les enzymes oxydases.

25. Procédé de teinture des fibres kératiniques et en particulier des fibres kératiniques humaines telles que les cheveux, **caractérisé par le fait qu'**on applique sur lesdites fibres au moins une composition selon l'une des revendications 13 à 23, pendant une durée suffisante pour développer la coloration désirée en présence d'un agent oxydant, l'agent oxydant étant appliqué avant, simultanément ou après la composition.

26. Procédé de teinture des fibres kératiniques et en particulier des fibres kératiniques humaines telles que les cheveux, **caractérisée par le fait qu'**on applique sur lesdites fibres la composition prête à l'emploi selon la revendication 24, pendant une durée suffisante pour développer la coloration désirée

27. Utilisation d'une composition comprenant, dans un milieu approprié pour la teinture, au moins un composé de formule générale (I) telle que défini à l'une des revendications 1 à 10 pour la teinture des fibres, de préférence les fibres kératiniques telles que les cheveux.

28. Utilisation de la composition selon la revendication 27 dans un dispositif à plusieurs compartiments, un premier compartiment contenant la composition cosmétique pour la teinture des fibres kératiniques et un deuxième compartiment contenant un agent oxydant.
